Europäisches Patentamt

European Patent Office

Office européen des brevets

(11) Publication number: **0 386 240 B1**

# EUROPEAN PATENT SPECIFICATION

(45) Date of publication of patent specification: **19.07.95**  (51) Int. Cl.⁶: **C12N 9/64**, C12N 15/58, A61K 38/49

(21) Application number: **88909384.5**

(22) Date of filing: **28.10.88**

(86) International application number:
**PCT/JP88/01098**

(87) International publication number:
**WO 89/03874 (05.05.89 89/10)**

(54) **NOVEL POLYPEPTIDE COMPOUNDS.**

(30) Priority: **29.10.87 JP 274770/87**
**18.12.87 JP 320785/87**
**23.03.88 JP 70126/88**
**27.07.88 JP 189351/88**

(43) Date of publication of application:
**12.09.90 Bulletin 90/37**

(45) Publication of the grant of the patent:
**19.07.95 Bulletin 95/29**

(84) Designated Contracting States:
**AT BE CH DE FR GB IT LI LU NL SE**

(56) References cited:
**EP-A- 231 624**
**EP-A- 0 233 013**
**EP-A- 0 241 208**
**WO-A-86/01538**
**JP-A- 6 248 378**

**See also references of WO8903874**

(73) Proprietor: **YAMANOUCHI PHARMACEUTICAL CO. LTD.**
**No. 3-11 Nihonbashi-Honcho, 2-chome**
**Chuo-ku**
**Tokyo 103 (JP)**

(72) Inventor: **KAWAUCHI, Yasushi**
**16-1, Hasune 3-chome**
**Itabashi-ku**
**Tokyo 174 (JP)**
Inventor: **TAKEMOTO, Toshiyuki**
**3-4-607, Tate 2-chome**
**Shiki-shi**
**Saitama 353 (JP)**
Inventor: **TAKAYAMA, Makoto**
**3-14, Nishi Ohi 5-chome**
**Shinagawa-ku**
**Tokyo 140 (JP)**
Inventor: **YOKOTA, Masami**
**5-21, Oomori Nishi 5-chome**
**Oota-ku**
**Tokyo 143 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Nature, vol. 301, (1983), Diane Pennica et al. p. 214-221**

**Proc. Natl. Acad. Sci. USA, vol. 83, no. 13, (1986) Anton-Jan van Zonneveld et al. pp. 4670-4674**

**The Journal of Biological Chemistry, vol. 261, no. 30, (1986), pp. 14214-14218**

Inventor: **KATO, Masao**
**65-1-305, Harigaya**
**Fujimi-shi**
**Saitama 354 (JP)**
Inventor: **KATSUTA, Kimio**
**1501, Kubota**
**Yoshimi-cho**
**Hiki-gun**
**Saitama 355-01 (JP)**
Inventor: **GUSHIMA, Hiroshi**
**22-8, Asamadai 3-chome**
**Ageo-shi**
**Saitama 362 (JP)**

(74) Representative: **Geering, Keith Edwin et al**
**REDDIE & GROSE**
**16 Theobalds Road**
**London WC1X 8PL (GB)**

## Description

(FIELD OF THE INVENTION)

The present invention relates to a novel improved tissue plasminogen activator (improved t-PA) having a prolonged biological half life and increased stability to heat and acids and which has an inhibitory effect against inflammation around the thrombus-formed region.

The present invention further includes deoxyribonucleic acid (DNA) coding for the said improved t-PA, a recombinant expression vector containing DNA encoding the improved t-PA, host cells transformed by said expression vector, a process for production of the improved t-PA, a drug composition comprising the improved t-PA and its use for treatment of thrombotic disease.

(BACKGROUND OF THE INVENTION)

It is known that human tissue plasminogen activator (t-PA) possesses useful fibrinolytic activity in extremely efficiently activating fibrin-bound plasminogen, while it does not efficiently activate plasminogen in the circulating body fluid phase in contrast to ordinary thrombolytic agents, streptokinase (SK) and urokinase (UK). The amino acid sequence constituting the human t-PA and nucleotide sequence of cDNA coding for human t-PA are known [Pennica, D., et al., Nature, 301, 214-221 (1983)]. It is also known that human t-PA dissolves venous and arterial blood clots. In large scale clinical tests, it is reported that human t-PA given intravenously is effective for re-perfusion of obstructive coronary artery in patients with acute myocardial infarction.

However, a defect in applying this human t-PA to the treatment of thrombotic disease is the extremely short half life of its enzyme activity in blood [Rijken, D.C., et al., Thromb. Heamost., 54 (1), 61 (1985), Hubert, E.F., et al., Blood, 65, 539 (1985)]. Accordingly, when used for the treatment of thrombotic disease, human t-PA should be continuously administered intravenously in a high dose.

It is known that naturally occurring human t-PA takes a domain structure, from the N-terminal of the molecule, of the finger domain, the EGF (epidermal growth factor) domain, the two domains of kringle 1 and kringle 2 and the serine protease domain, based on its fully anticipated secondary structure. It is reported by Rijken et al. [Rijken, D.C., et al., Thromb. Haemost., 54 (1), 61 (1985)] that domains of human t-PA other than the serine protease domain would be responsible for the shortness of biological half life of human t-PA. It is also reported by Zonneveld et al. [Zonneveld, A.J.V., et al., Proc. Natl., Acad. Sci. U.S.A., 83, 4670 (1986)] that the finger domain, the EGF domain and the kringle 2 domain structure would take an important role for fibrin binding activity of naturally occurring human t-PA and for maintaining fibrin-dependent activation of t-PA. However, no concrete measure for maintaining fibrin binding activity possessed by naturally occurring human t-PA and desirable properties of fibrin-dependent activity and prolonging the biological half life is known.

In Published Unexamined Japanese Patent Application Laid Open No. 48378/1987, there is described t-PA obtained by deletion of 87th to 175th amino acids of naturally occurring human t-PA in which kringle 1 is deleted. This t-PA is characterized by further site mutation in the EGF region. The Japanese Patent Application discloses that the modified t-PA has a binding ability to fibrin and interaction with a tissue plasminogen activator inhibitor is reduced.

In EP No. 241208, there is described t-PA obtained by deletion of 92nd to 179th amino acids of naturally occurring human t-PA in which kringle 1 is deleted. It is simply mentioned that this t-PA has a fibrinolytic activity.

Furthermore, EP No. 231624 discloses modified t-PA showing a prolonged half life. The modified t-PA having F-EGF-K2-A as one of its sequence is deleted of kringle 1 but no specific process for its production is shown. It is understood that the modified t-PA in the present invention is different from naturally occurring t-PA in the amino acid sequence in the inter-domain region, in light of the specifically recited process therefor.

As a result of extensive investigations, the present inventors have produced an improved t-PA which contains the finger domain, the EGF domain, the kringle 2 domain and the serine protease domain but in which the first kringle 1 domain is deleted at a specific amino acid site and which has site-specific mutation at the kringle 2 domain-serine protease linking site; this improved t-PA has excellent stability to heat and acids, has markedly prolonged biological half life and also has antiinflammatory activity, while unexpectedly maintaining desirable properties of naturally occurring human t-PA.

(DISCLOSURE OF THE INVENTION)

The present invention relates to an improved t-PA. The improved t-PA of the present invention is quite different in its chemical structure from naturally occurring human t-PA and exhibits more excellent functions.

The improved t-PA of the present invention is a polypeptide having the following amino acid sequence in which, compared to human t-PA, kringle 1 is deleted and Arg 275 is substituted by Glu :

```
H₂N — Ser Tyr Gln Val Ile Cys Arg Asp Glu Lys
       Thr Gln Met Ile Tyr Gln Gln His Gln Ser
       Trp Leu Arg Pro Val Leu Arg Ser Asn Arg
       Val Glu Tyr Cys Trp Cys Asn Ser Gly Arg
       Ala Gln Cys His Ser Val Pro Val Lys Ser
       Cys Ser Glu Pro Arg Cys Phe Asn Gly Gly
       Thr Cys Gln Gln Ala Leu Tyr Phe Ser Asp
       Phe Val Cys Gln Cys Pro Glu Gly Phe Ala
       Gly Lys Cys Cys Glu Ile Asp Thr Arg Ala
       Thr Ser Glu Gly Asn Ser Asp Cys Tyr Phe
       Gly Asn Gly Ser Ala Tyr Arg Gly Thr His
       Ser Leu Thr Glu Ser Gly Ala Ser Cys Leu
       Pro Trp Asn Ser Met Ile Leu Ile Gly Lys
       Val Tyr Thr Ala Gln Asn Pro Ser Ala Gln
       Ala Leu Gly Leu Gly Lys His Asn Tyr Cys
       Arg Asn Pro Asp Gly Asp Ala Lys Pro Trp
       Cys His Val Leu Lys Asn Arg Arg Leu Thr
       Trp Glu Tyr Cys Asp Val Pro Ser Cys Ser
       Thr Cys Gly Leu Arg Gln Tyr Ser Gln Pro
       Gln Phe  Y  Ile Lys Gly Gly Leu Phe Ala
       Asp Ile Ala Ser His Pro Trp Gln Ala Ala
       Ile Phe Ala Lys His Arg Arg Ser Pro Gly
       Glu Arg Phe Leu Cys Gly Gly Ile Leu Ile
       Ser Ser Cys Trp Ile Leu Ser Ala Ala His
       Cys Phe Gln Glu Arg Phe Pro Pro His His
       Leu Thr Val Ile Leu Gly Arg Thr Tyr Arg
       Val Val Pro Gly Glu Glu Glu Gln Lys Phe
       Glu Val Glu Lys Tyr Ile Val His Lys Glu
```

4

```
Phe Asp Asp Asp Thr Tyr Asp Asn Asp Ile
Ala Leu Leu Gln Leu Lys Ser Asp Ser Ser
Arg Cys Ala Gln Glu Ser Ser Val Val Arg
Thr Val Cys Leu Pro Pro Ala Asp Leu Gln
Leu Pro Asp Trp Thr Glu Cys Glu Leu Ser
Gly Tyr Gly Lys His Glu Ala Leu Ser Pro
Phe Tyr Ser Glu Arg Leu Lys Glu Ala His
Val Arg Leu Tyr Pro Ser Ser Arg Cys Thr
Ser Gln His Leu Leu Asn Arg Thr Val Thr
Asp Asn Met Leu Cys Ala Gly Asp Thr Arg
Ser Gly Gly Pro Gln Ala Asn Leu His Asp
Ala Cys Gln Gly Asp Ser Gly Gly Pro Leu
Val Cys Leu Asn Asp Gly Arg Met Thr Leu
Val Gly Ile Ile Ser Trp Gly Leu Gly Cys
Gly Gln Lys Asp Val Pro Gly Val Tyr Thr
Lys Val Thr Asn Tyr Leu Asp Trp Ile Arg
Asp Asn Met Arg Pro-COOH
```

wherein Y represents Glu, H$_2$N- represents the amino terminal, and -COOH represents the carboxy terminal.

This t-PA of the present invention is termed herein "(improved) t-PA (VI)"; it is illustrated herein partly by comparison reference to t-PA (II) and t-PA (V) which are not according to the invention.

The improved t-PA (VI) found by the present inventors is excellent in stability to heat and acids, has markedly prolonged biological half life and also has an antiinflammatory activity, as will later be described, while maintaining desirable properties of naturally occurring human t-PA.

The present invention also aims at expressing the improved t-PA utilizing recombinant DNA manipulation. The present invention also provides novel DNA compounds coding for the improved t-PA and recombinant DNA expression vectors. The present invention also provides host cells transformed with novel cloning vectors. By the use of the DNA compounds, t-PA derivatives the presence of which is unknown so far in the natural world can be produced.

(BRIEF DESCRIPTION OF THE DRAWINGS)

Fig. 1 shows a base sequence of 16 oligodeoxynucleotides used for construction of synthetic gene fragment coding for t-PA [II].

Fig. 2 shows a synthetic gene fragment for construction of t-PA [II] containing restriction enzymes Bgl II and Eco RI ends at both termini, which is constructed using 16 oligodeoxynucleotides shown in Fig. 1.

Fig. 3-1 shows the procedure for constructing t-PA [II]. In the figure, the black portion, oblique portion and white-on-black portion denote a region encoding mature t-PA protein, a region encoding prepropeptide and a non-translation region, respectively.

Fig. 3-2 shows a method for verification of synthetic gene fragment block IV through determination of DNA base sequence by the dideoxy method and the 7-DEAZA method.

Fig. 4 shows the procedure for constructing expression vector pVYI in animal cells and integration of improved t-PA into pVYI.

Fig. 5-1 and Fig. 5-2 show DNA base sequences encoding t-PA [II] and t-PA [V ], respectively.

Fig. 6-1 and Fig. 6-2 show amino acid sequences derived from DNA base sequences encoding t-PA [II] and t-PA [V ], respectively.

Fig. 7 shows restriction enzyme and function map of plasmid pTPA2 having Eco RI-Xho II fragment (about 1,000 base pairs) of naturally occurring t-PA gene integrated into vector pBR322 at the cleavage sites with Eco RI and Bam HI.

Fig. 8 shows mp9 (t-PA [II]) having Bgl II-Xho II fragment (about 1,500 base pairs) of t-PA [II] gene integrated into double stranded M13mp9 DNA at the cleavage site with Bam HI.

Fig. 9 shows the dose-response curve of the t-PA activity of improved t-PA [VI] and naturally occurring t-PA by the S-2251 method in the presence (+Fb) and absence (-Fb) of a fibrin substitute.

Fig. 10 shows change in activity of improved t-PA [VI] and naturally occurring t-PA in rabbit blood with passage of time.

Fig. 11 shows change in residual activity of improved t-PA [VI] after heat treatment.

Fig. 12 shows inhibition of improved t-PA [VI] against LAF activity.

Fig. 13 shows activated state of improved t-PA [VI] with denatured protein.

Fig. 14 shows degradation of denatured protein with improved t-PA [VI].


(BEST MODE FOR PRACTICING THE INVENTION)


Hereafter the process for production, DNA compounds and transformed cells are described in detail.


(Process for production of improved t-PA)


The gene coding for the t-PA molecule used to produce the improved t-PA molecule of the present invention was obtained from a cDNA bank prepared from Bowes human melanoma cells. Poly(A)$^+$ RNA was isolated from Bowes human melanoma cells and fractionated by sucrose density gradient centrifugation. Next, a small amount of the fractionated poly(A)$^+$ RNA was taken and the mRNA fraction encoding t-PA gene was identified by the dot hybridization method using oligonucleotide probe capable of recognizing the specific sequence of t-PA mRNA. Using this t-PA mRNA-rich fraction as a starting material, the cDNA bank was prepared and screened by the probe used for the identification of t-PA mRNA described above. Since no clone having the complete t-PA gene sequence was isolated, the remaining base sequence required for constructing the improved t-PA gene was synthesized with a DNA synthesizer to construct the objective gene. Then, the objective gene was constructed by the site-specific mutation induction method.

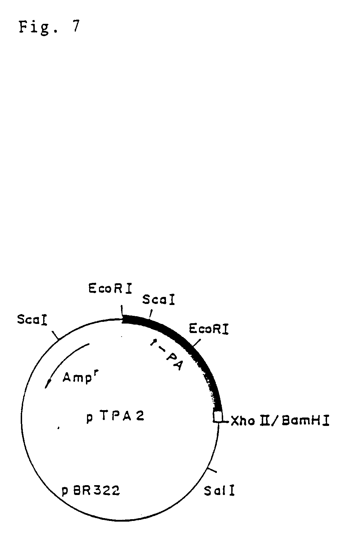

The Eco RI-Xho II fragment of naturally occurring t-PA gene (about 1,000 base pairs), a part of which was deleted at the N-terminal was introduced into vector pBR322 at the cleavage sites with Eco RI and Bam HI to construct pTPA2. A strain (E. coli HB 101/pTPA2) obtained by transforming E. coli with this plasmid has been deposited in the Fermentation Research Institute of the Agency of Industrual Science & Technology of Japan under Registration No. P-9649 (FERM BP-2107). Restriction enzyme and function map of plasmid pTPA2 is shown in Fig. 7.

Next, this improved t-PA gene was inserted into plasmid pVYI.

Plasmid pVYI is obtained by ligating about 2,900 base pair Bam HI-Kpn I fragment of plasmid pKSV10 (manufactured by Pharmacia Fine Chemicals) with Eco RI cleavage fragment of plasmid pAdD26SV(A) No. 3 (N) (obtained from Dr. Hiroshi Handa of Tokyo University), after rendering both ends blunt. Accordingly, this vector contains mouse dihydrofolate reductase cDNA gene under transcription control of adenovirus (Ad2) major late promoter, SV40 early promoter upstream the improved t-PA gene inserted site and, intervening sequence or intron and polyadenylation sequence downstream.

The gene of the present invention contains genetic information for producing the improved t-PA. Therefore, the gene is integrated into an appropriate expression vector, and the integrated expression vector is introduced into a suitable host cell to prepare transformants, whereby expression is effected. Thus, the improved t-PA can be produced by means of bioengineering. As host cells, prokaryotic cells such as E. coli, Bacillus subtilis, etc., eukaryotic microorganisms such as yeast, etc. and higher animal cells can be used. As E. coli, JM109 strain, W3110 Q strain, etc. belonging to K12 strain are generally used; as Bacillus subtilis, BD170 strain, BR151 strain, etc. are used. As yeast, RH218 strain, SHY1 strain, etc. of Saccharomyces cerevisiae can be utilized.

For expression with host cells, plasmid vector or phage vector containing replicon derived from species compatible with host cells and regulatory sequence are generally used. Examples of the vector for E. coli as the host cell include plasmids such as pBR322, pUC18, pUC19, etc.; λ phage such as λgt, Charon 4A, etc.; M13 phage, and the like. As the vector for Bacillus subtilis, pUB110, pSA2100, etc. can be used and as the vector for yeast, YRp7, YEp61, etc. can be used.

The vector must bear a promoter capable of expressing the objective protein. As the promoter for E. coli gene or phage gene, for example, lac, trp, tac, trc, PL, etc. can be utilized.

Culture cells of higher animals for use as the host are kidney cells of rhesus monkey, mosquito larva cells, kidney cells of African green monkey, mouse fetal fibroblast, Chinese hamster ovary cells, human fetal kidney cells, moth oval tissue cells, human cervical epithelium-like cells, human myeloma cells, mouse fibroblasts, etc. As the vector, there can be used SV40 early promoter, SV40 late promoter, SV40 bearing a promoter from eukaryotic gene (for example, estrogen-inducing avian ovalbumin gene, interferon gene, glucocorticoid-inducing tyrosine aminotransferase gene, thymidine kinase gene, adenovirus early and late genes, phosphoglycerate kinase gene, $\alpha$ factor gene, etc.), bovine papilloma virus or derivative vectors thereof and the like.

It is further known that t-PAs secreted and produced by cells have various N-termini, depending upon difference in cleavage sites.

As the N-terminal sequences that are generally known, there are the amino acid sequence of the present invention in which 3 amino acids (Ser Tyr Gln) at the N-terminal end are further cleaved and the N-terminal begins with Val or in which Ser is preceded by Gly Ala Arg at the N-terminal; and the like.

As such, in the case of secreting and producing t-PA using culture cells as the host, the way of cleavage with signal peptidase or protease varies depending upon the kind of cells so that t-PA species having different N-termini may also be produced.

This phenomenon applies not only to the case of secretion and production by culture cells but it is considered that a similar phenomenon would also occur with the N-terminal of t-PA produced by E. coli, Bacillus subtilis, yeast, and other cells.

For transformation of the host using the expression vector having integrated therein the improved t-PA gene, the Hanahan method [Hanahan, D., J. Mol. Biol., 166, 557 (1983)] can be adopted in the case of E. coli; in the case or animal cells, the calcium phosphate method [Van der Eb, A.J. and Graham, F.L., Method in Enzymology, 65, 826 (1980), academic Press], etc. can be adopted.

The DNA sequence and plasmid explained in the present invention can be subjected to various modifications and variations. For example, it is possible to substitute the nucleotide over the entire regions encoding polypeptide because of synonymity of the gene; at the same time, it is also possible to replace

```
T A A       T A G
| | | or | | |
A T T       A T C
```

translation termination signal for

```
T G A
| | |
A C T
```

translation termination signal specifically exemplified. Such a sequence can be presumed from the amino acid or DNA sequence of human t-PA currently known and can be constructed by conventional DNA synthesis method. Accordingly, the base sequence of the gene of the present invention is not limited to only one DNA sequence in any sense.

As described above, the improved t-PA is useful for the treatment of various acquired diseases involving vascular coagulation including deep vein, pulmonary arterial embolism, peripheral arterial thrombosis, heart or peripheral artery-derived embolism, acute myocardial infarction and thrombotic attack.

Like naturally occurring human t-PA, the improved t-PA is particularly useful for treatment of acute myocardial infarction. As has recently be proven, naturally occurring human t-PA is effective for dissolution of occlusive coronary arterial thrombus, regeneration of myocardiac perfusion and recovery of most parts in ischemic myocardiac layer, when intravenously administered in a dose of 30 to 70 mg over 1 to 3 hours. The improved t-PA has a markedly prolonged half life in blood and is thus effective as in naturally occurring human t-PA. It is expected that the improved t-PA would, be as effective clinically as with naturally occurring human t-PA, in a dose of about 10% of the dose recommended with naturally occurring human t-PA even in a single administration.

In addition, the improved t-PA of the present invention further exhibits the following useful properties that are unknown so far with naturally occurring human t-PA and modified t-PAs.

a) Antiinflammatory activity

In the thrombus region, not only formation of the thrombus but also formation of fibrin degradation products or a trace amount of kinin, etc. are recognized. It is known that these substances have an inflammation-inducing activity and thus cause inflammation in the thrombus region. For such a reason, it is desired that a thrombolytic agent used for treatment of thrombosis should possess not only a thrombolytic activity but also an antiinflammatory activity.

As a result of extensive investigations, the present inventors have succeeded in imparting antiinflarnmatory activity based on two functions to the improved t-PA.

One is the experimental fact that the improved t-PA inhibits the biological activity of interleukin 1 (IL-1) which is one mediator for inflammatory reaction. IL-1 produced in macrophage is considered to participate in inflammatory reaction via pyrexia, acceleration of growth of fibroblast, production of colagenase in synovial cell membrane, etc., or acceleration of synthesis of prostacycline in vascular endothelial cells. It is also known that IL-1 acts on liver cells to accelerate the production of proteins (serum amyloid protein, fibrinogen, etc.) in the acute phase which increases upon inflammation. Now the present inventors have found that the improved t-PA inhibits the activity (LAF activity) of enhancing mitogen reactivity of mouse thymocyte, which is one of the biological activities of IL-1.

The other is that the improved t-PA has affinity to denatured protein (denatured IgG, denatured albumin, etc.) caused by inflammation in the thrombus region and additionally possesses the property of being activated by the denatured protein.

By this activity, the improved t-PA does not act on protein that undergoes no denaturation but decomposes only denatured protein in the inflammatory region, and inflammation can be palliated. The present inventors have confirmed by SDS gel electrophoretic analysis that the improved t-PA decomposes the denatured protein alone.

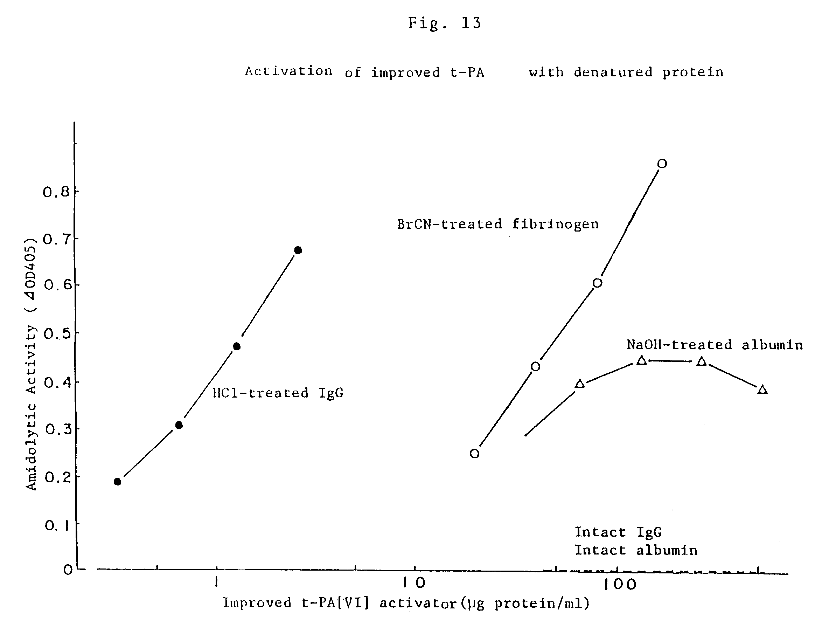

As shown in Fig. 13, the activation activity and selectivity of the improved t-PA by denatured protein are remarkable. In HCl-treated IgG, similar activity was shown in a concentration reduced by one-several tenth of BrCN-treated fibrinogen. On the other hand, normal IgG did not develop the activation action to the improved t-PA even in a concentration of 500 $\mu$g/ml.

b) Prevention of reocclusion after reperfusion of occluded blood vessel

It is known that when thrombosis was treated by natural t-PA, reocclusion was noted with high frequency after reperfusion of the occluded blood vessel. For this reason, combined therapy with a platelet coagulation inhibitor or an anticoagulant has been performed in practice. However, the combined therapy involves problems of interaction of drugs, dose control, side effects, etc. Rather, it is more desired that t-PA itself additionally possesses the activity of preventing reocclusion

The improved t-PA of the present invention has per se the activity of preventing reocclusion, based on the mechanism of two types of activity.

1) The first is preventing rapid reduction in t-PA concentration after administration of the improved t-PA (due to its prolonged durability), thereby getting rid of the rebound phenomenon and thus preventing occurrence of reocclusion.

2) The second is preventing impairment of vascular endothelial cells caused by IL-1, so that platelet coagulation is indirectly inhibited to prevent occurrence of reocclusion.

c) Enhanced stability

Protein preparations are generally unstable so that it is required to preserve the preparations in a freeze dried state or at low temperatures in a solution state. It is expected that a plasminogen activator is administered to the patient with acute myocardiac infarction and in this case, it is considered to be necessary that the plasminogen activator should be administered within several hours after the onset of attack, in order to reduce the mortality rate. Therefore, the plasminogen activator preparations should be stored in various places but depending upon place, it may occur that facilities for storing at low temperature might not be utilizable. In such a case, stable preparations that can be stored at room temperature are desired.

Further when the stability is improved, it is possible to perform heat treatment, treatment with acids, etc. during the course of making preparations. Particularly in the improved t-PA of the present invention which is produced by cell culture, it becomes possible to remove cell-derived retrovirus known to be weak against heat.

Hereafter the present invention is described more specifically with reference to the examples below but is not deemed to be limited thereto. Unless otherwise indicated, recombinant DNA is produced by the laboratory manual described below.

Maniatis, T., et al., Molecular Cloning: A Laboratory Manual, Cold Spring Harbor Laboratory, Cold Spring Harbor, NY (1982)

Example 1. Cloning of t-PA cDNA

Bowes human melanoma cells (acquired from Dr. Roblin, R. in the National Cancer Institute, USA) are cultured in a manner similar to the method of Opdenakker et al. [Opdenakker, G., et al., Eur. J. Biochem., 131, 481-487 (1983)]. In order to induce t-PA mRNA, TPA (12-O-tetradecanoylphorbol 13-acetate) is added to the culture in the final concentration of 100 ng/ml followed by culturing for 16 hours. Next, the total cell RNA is extracted from the culture cells according to the modified method of Freeman et al. [Okayama/Berg cDNA Manual, page 3 (1985), Pharmacia Fine Chemicals]. Using oligo-dT cellulose column (manufactured by Pharmacia Fine Chemicals), poly(A)$^+$ RNA is separated from the total cell RNA. As a result, about 400 $\mu$g of poly(A)$^+$ RNA is obtained from approximately $10^9$ cells.

This poly(A)$^+$ RNA is fractionated by sucrose density gradient centrifugation in conventional manner. A part of the fractionated poly(A)$^+$ RNA is taken and dot blot hybridization [Perbal, B., A Practical Guide to Molecular Cloning, 410 (1984), John Wiley & Sons, Inc.] is performed using oligonucleotide probe specific to t-PA mRNA to estimate the t-PA mRNA fraction. The probe (probe Y) used in this case has a base sequence of 5'-GCTTGGCAAAGATGGCA-3' which is complementary to the mRNA region encoding the +291 to +297 amino acid sequence of t-PA reported by Pennica et al. supra and synthesized by the $\beta$-cyanophosphamidide method using Model 380A DNA Synthesizer (manufactured by Applied Biosystems). Synthesis of DNA oligomer, removal of protection group, cleavage from resin and purification are carried out as instructed by the manual for Model 380A DNA Synthesizer. Radioactive labelling of probe Y at the 5'-terminal is performed according to the Laboratory Manual, page 122, using T4 polynucleotide kinase (manufactured by Takara Shuzo Co., Ltd.) and $\gamma$-[$^{32}$P] ATP.

Probe Y is strongly hybridized mainly with 20 to 30S poly(A)$^+$ RNA (this fraction is called Fraction M).

Using as a template 10 $\mu$g of poly(A)$^+$ RNA obtained from Fraction M, 3 $\mu$g of double stranded cDNA is synthesized using reverse transcriptase (manufactured by Biochemical Industry Co., Ltd.) according to the Gubler-Hoffman method [Gubler, U. and Hoffman, B.J., Gene, 25, 263 (1983)] and deoxy C chain is added to the double stranded cDNA at the 3'-terminal thereof according to the method of Deng-Wu [Deng, G.R. and Wu, R., Nucleic Acids Res., 9, 4173 (1981)]. Next, the deoxy C chain-added double stranded cDNA is subjected to gel filtration on CL4B Sepharose (manufactured by Pharmacia Fine Chemicals) to remove low molecular nucleic acids having base pairs less than about 500. Thereafter, the cDNA is subjected to annealing with pBR322 (manufactured by Bethesda Research) with added deoxy G chain at the Pst I site in conventional manner. Using the mixture resulting after the annealing, E. coli HB101 competent cells (manufactured by Takara Shuzo Co., Ltd.) are transformed. As a result, a cDNA bank composed of about 40,000 independent transformants is obtained.

This cDNA is subjected to colony hybridization using probe Y described above according to the method of Woods [Woods, D., Focus, 6(3), 1 (1984) manufactured by Bethesda Research Lab.] to obtain clones reacted with probe Y. Among the clones, a base sequence of the cDNA region is determined with respect to plasmid pTPA1 of clone containing the longest t-PA cDNA. The method followed the dideoxy method [Carlson, J., et al., J. Biotechnology, 1, 253 (1984)] using M13 phage vector and the 7-DEAZA method [Mizusawa, S., et al., Nucleic Acids Res., 14, 1319 (1986)]. As a result, it is noted that plasmid pTPA1 contained the base sequence from T at +441 to A at +2544 for t-PA gene reported by Pennica et al. supra.

Example 2. Construction of t-PA [II]

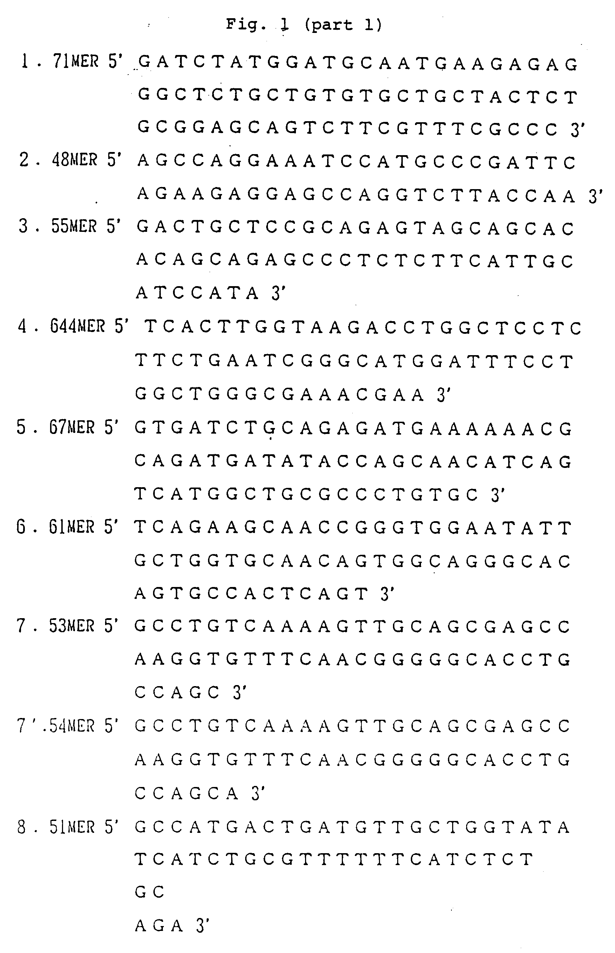

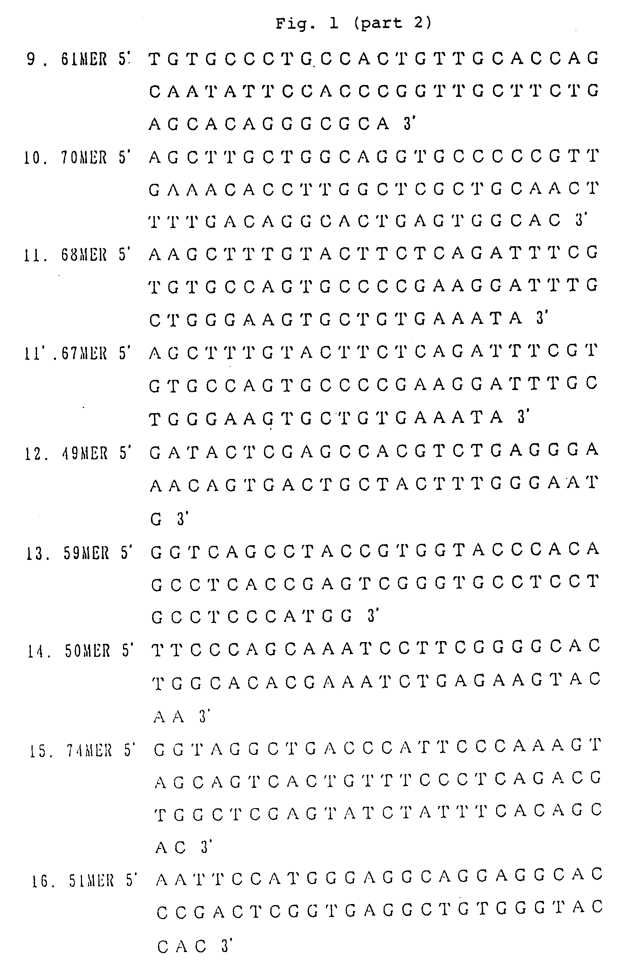

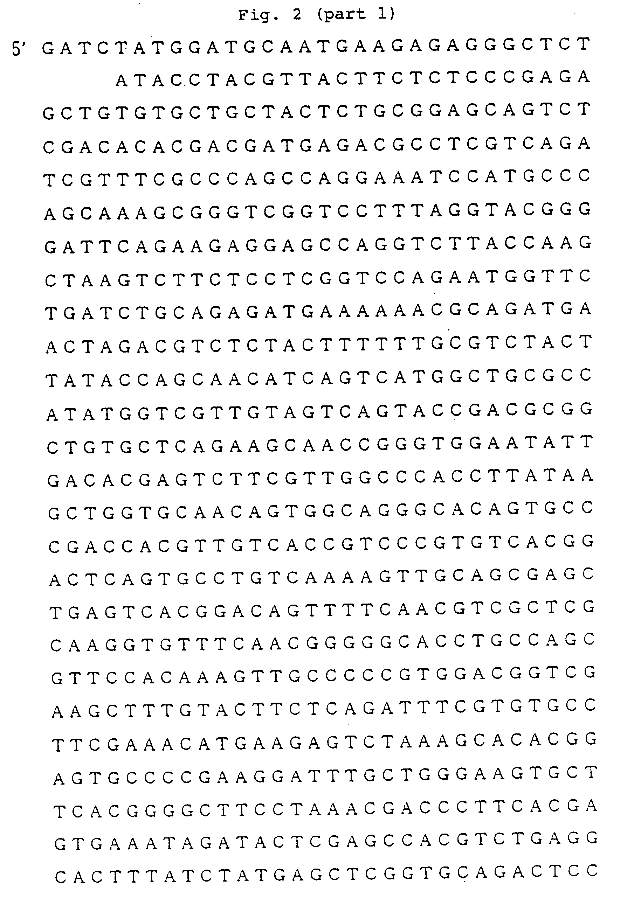

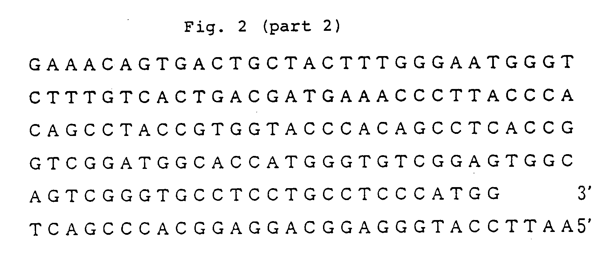

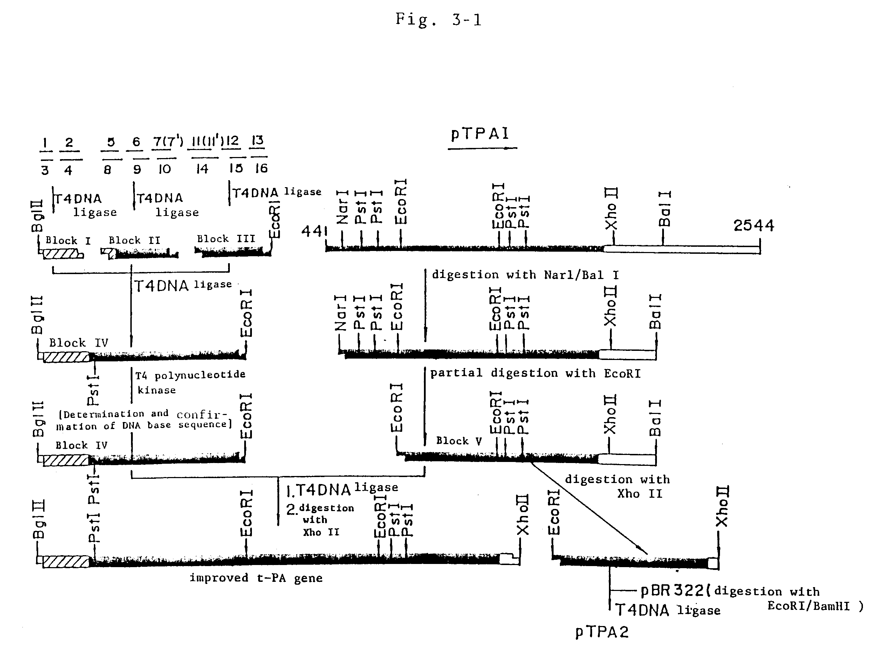

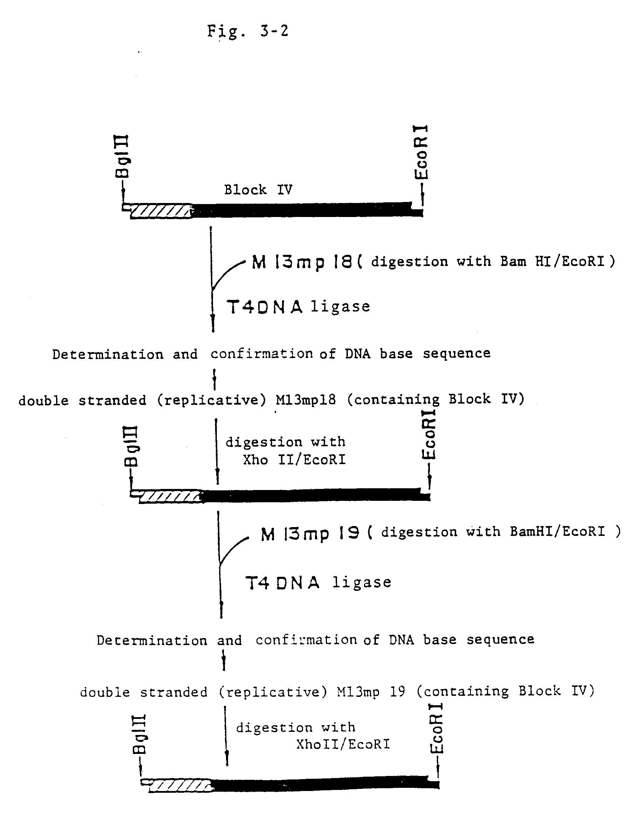

In plasmid pTPA1 shown in Example 1, the N-terminal region is insufficient to construct t-PA ([II] which is deleted of the kringle 1 domain. Therefore, the insufficient DNA segment is synthesized as described above using 380A DNA Synthesizer (manufactured by Applied Biosystems). The base sequence of the synthesized oligomer and the entire synthesized sequence are shown in Figs. 1 and 2, respectively. Furthermore, specific procedures for construction of t-PA [II] using these oligomers are shown in Figs. 3-1 and 3-2, respectively.

2-1) Construction of block IV (Bgl II-Eco RI fragment, about 480 base pairs)

Block IV fragment in Fig. 3-1 is prepared as follows.

Firstly, according to the Laboratory Manual, page 122, 40 pmoles each of synthetic oligonucleotides 2, 3, 4, 5, 6, 7, (7'), 8, 9, 10, 11, (11'), 12, 13, 14 and 15 shown in Fig. 1 is phosphorylated with 10 units of T4 polynucleotide kinase (manufactured by Takara Shuzo Co., Ltd.) at 37°C for an hour in 50 $\mu$l each of reaction solution. The reaction solution is treated with phenol. After ethanol precipitation, the precipitates are dried under reduced pressure and dissolved in sterile distilled water. After allowing 40 pmoles each of the oligomer in 150 $\mu$l of a solution containing 7 mM Tris-HCl (pH 7.5), 20 mM NaCl, 7 mM MgCl$_2$ and 0.1 mM EDTA to stand at 80°C for 5 minutes, at 60°C for 5 minutes and at room temperature for an hour in the respective blocks of block I (oligomers 1, 2, 3 and 4), block II (oligomers 5, 6, 7 (7'), 8, 9 and 10) and block III (oligomers 11, (11'), 12, 13, 14, 15 and 16), ethanol precipitation and drying under reduced pressure follow. The residue is dissolved in 40 $\mu$l of sterile distilled water. The reaction is carried out in 400 $\mu$l of the reaction solution at 4°C for 15 hours using DNA ligation kit (manufactured by Takara Shuzo Co., Ltd.). After ethanol precipitation and drying under reduced pressure, the residue is dissolved in sterile distilled water: in the case of block I (1), gel electrophoresis is carried out in a concentration of 5% polyacrylamide (Laboratory Manual supra, page 173) and the fragment of about 100 base pairs is separated and isolated in a conventional manner (Laboratory Manual supra, page 178); and in the case of block II (2) and block III (3), gel electrophoresis is carried out in a concentration of 3% agarose gel (LMP Agarose, manufactured by BRL) (Laboratory Manual supra, page 150) and the fragments of about 190 base pairs are separated and isolated by electric elution, respectively, (Laboratory Manual supra, page 164).

Next, 0.1 $\mu$g, 0.2 $\mu$g and 0.2 $\mu$g of the fragments of block I, block II and block III isolated, respectively, are ligated using the aforesaid DNA ligation kit. Then, gel electrophoresis is carried out in a concentration of 1.5% agarose to separate Bgl II-Eco RI fragment (block IV) of about 480 base pairs. Then, DNA is isolated from the agarose gel by electric elution. This DNA is further phosphorylated in 100 $\mu$l of the reaction solution at 37°C for an hour using 10 units of the aforesaid T4 polynucleotide kinase followed by phenol treatment, ethanol precipitation and drying under reduced pressure.

This synthetic gene segment and the base sequence of block IV are confirmed by determining the base sequence according to the dideoxy method using M13 phage vector and the 7-DEAZA method. Specific procedures are shown in Fig. 3-2. After the block IV Bgl II-Eco RI fragment described above is ligated with M13mp18 DNA (manufactured by Boehringer Mannheim-Yamanouchi Co., Ltd.) cleaved with restriction enzymes Bam HI (manufactured by Boehringer Mannheim-Yamanouchi Co., Ltd.) and Eco RI (manufactured by Boehringer Mannheim-Yamanouchi Co., Ltd.) using the DNA ligation kit described above, its base sequence is determined using M13 sequence kit (manufactured by Takara Shuzo Co., Ltd.) and 7-DEAZA sequence kit (manufactured by Takara Shuzo Co., Ltd.).

The restriction enzyme Bgl II cleavage site and the Bam HI cleavage site are ligated by isoshizomer arrangement via [Bam HI cleavage end

```
                    Xho II
                     ↓
              G    GATCT
                 ┊------┊
              C    CTAGA
                     ┊
```

Bgl II cleavage site] and the ligated fragment can be cleaved with restriction enzyme Xho II, whereby the original Bgl II cleavage end and the Bam HI cleavage end appear, respectively.

In order to determine the base sequence more precisely, M13mp18 (including block IV fragment) phage is further infected to E. coli JM109 according to the method of Messing [Messing, J., Methods in Enzymology, 101, 20-78 (1983)] and double stranded DNA (replication type) is then produced. After this DNA (50 $\mu$g out of the produced DNA) is cleaved with restriction enzymes Xho II (manufactured by Boehringer Mannheim-Yamanouchi Co., Ltd.) and Eco RI, gel electrophoresis is performed in 1.5% agarose concentration to separate about 480 base pairs of fragment (block IV). This DNA is recovered by electric elution. After ligating the recovered DNA with M13mp19 DNA (manufactured by Boehringer Mannheim-Yamanouchi Co., Ltd.) cleaved with restriction enzymes Eco RI and Bam HI in a manner similar to above using the DNA ligation kit, its base sequence is determined. As described above, it can be verified to have an accurate base sequence by determining both DNA sequences using M13mp18 and M13mp19. Furthermore, M13mp19 (including block IV) double stranded replicative DNA is produced by the method described above. After cleaving this DNA (50 $\mu$g out of the product produced) with restriction enzymes Eco RI and Xho II, 1.5% agarose gel electrophoresis is carried out to separate about 480 base pairs of fragment (block IV). DNA is isolated by electric elution.

2-2) Isolation of block V (Eco RI-Bal I fragment, about 1250 base pairs)

From clone pTPA1 obtained in Example 1, plasmid DNAs are produced in large quantities according to the method described in the Laboratory Manual supra at page 86, as shown in Fig. 3-1. After cleaving 70 μg of the DNAs with restriction enzymes Bal I (manufactured by Takara Shuzo Co., Ltd.) and Nar I (manufactured by Nippon Gene Co., Ltd.), 0.8% agarose gel electrophoresis is carried out to separate the Nar I-Bal I fragment (about 1540 base pairs). DNA is isolated by electric elution. After further partially digesting this DNA with restriction enzyme Eco RI, 0.8% agarose gel electrophoresis is carried out to separate the Eco RI-Bal I fragment (about 1250 base pairs). DNA is isolated by electric elution.

2-3) Construction of t-PA [II] gene from block V and block IV

As shown in Fig. 3-1, the t-PA [II] gene is produced as follows.

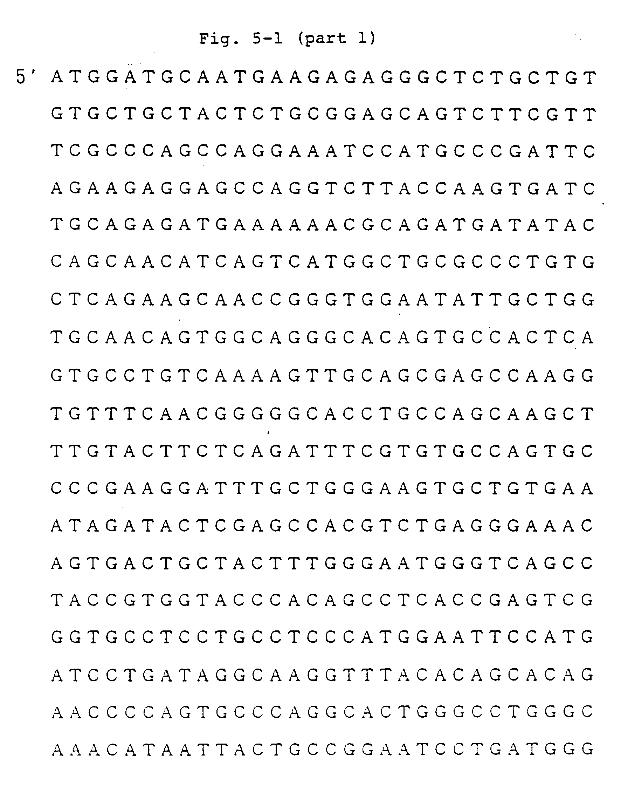

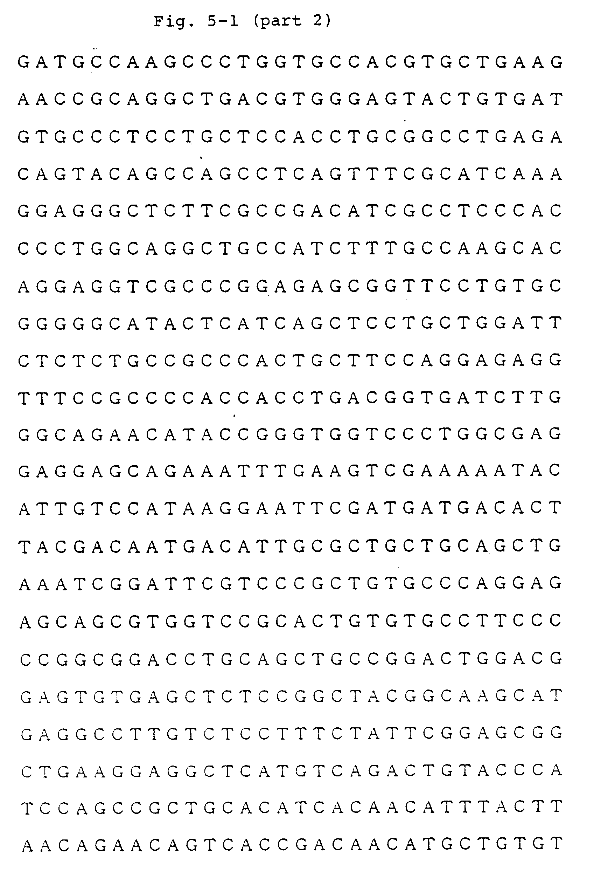

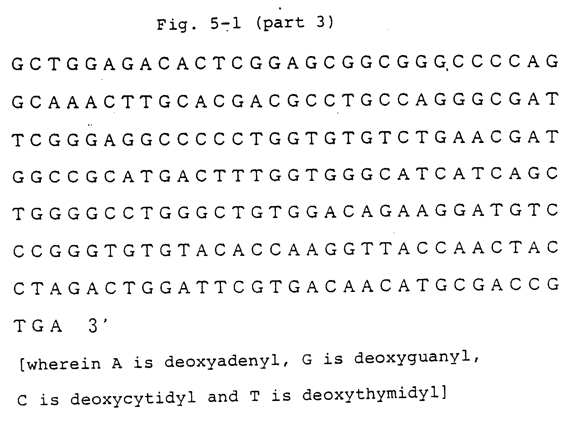

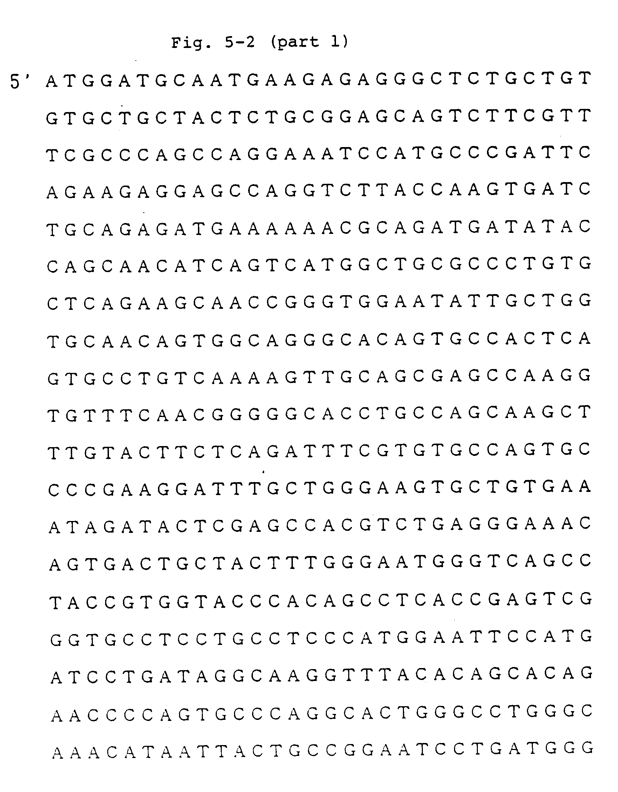

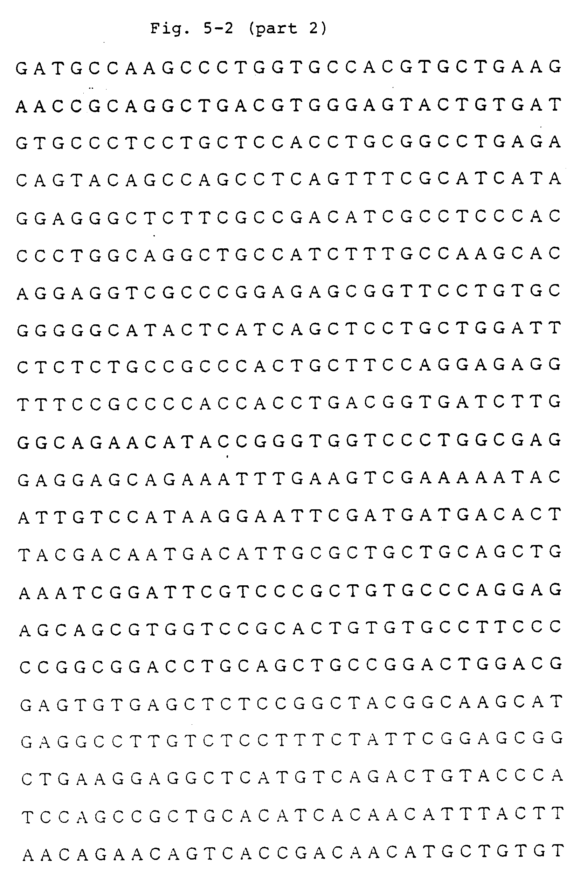

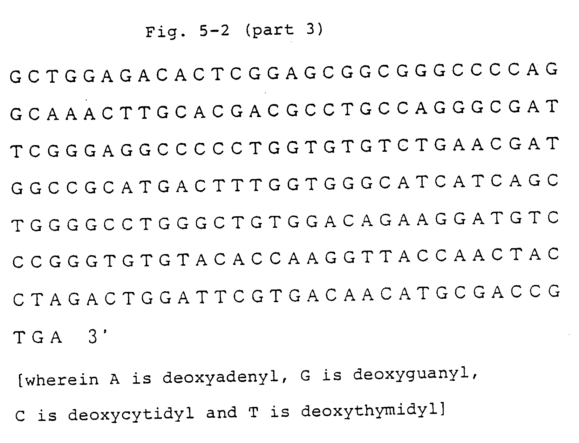

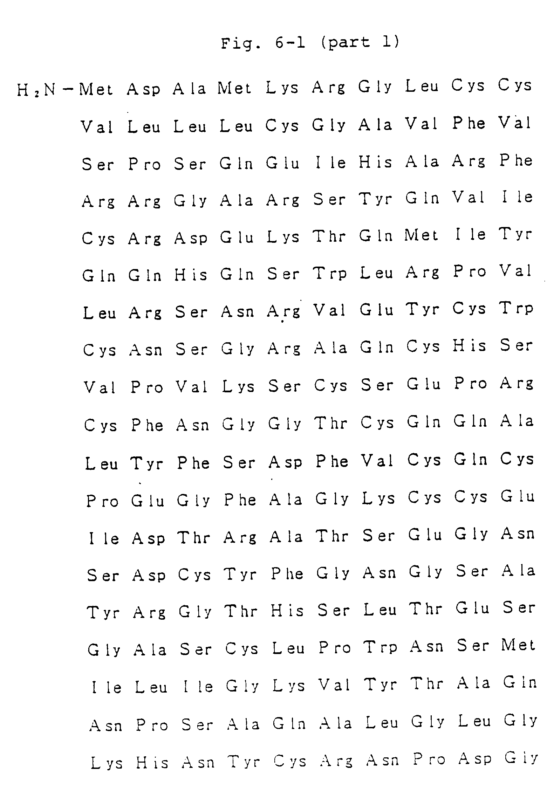

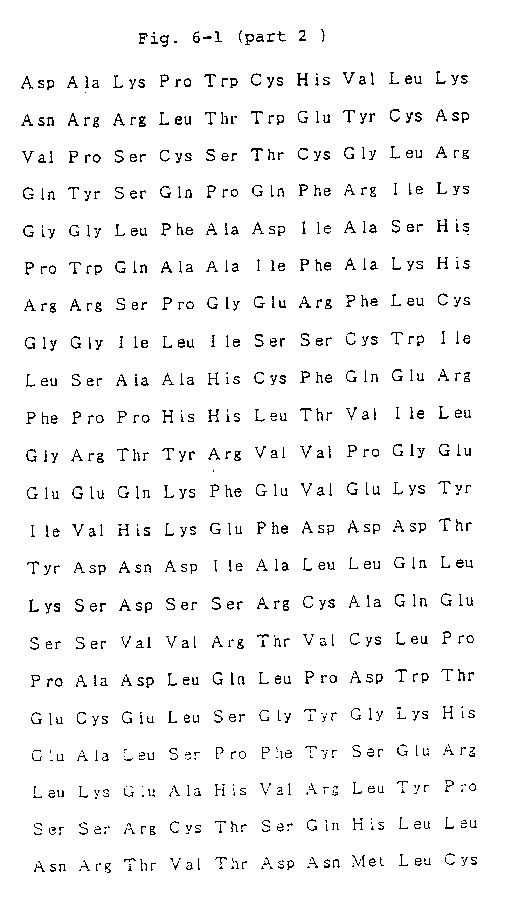

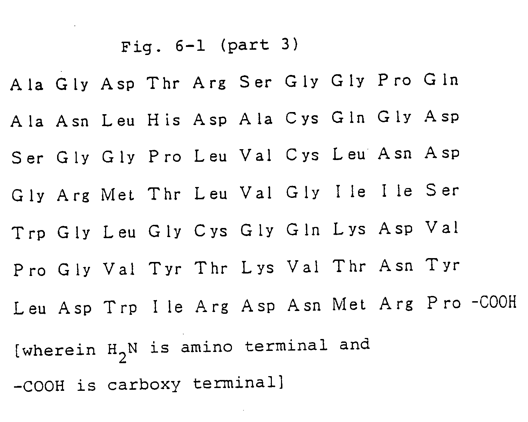

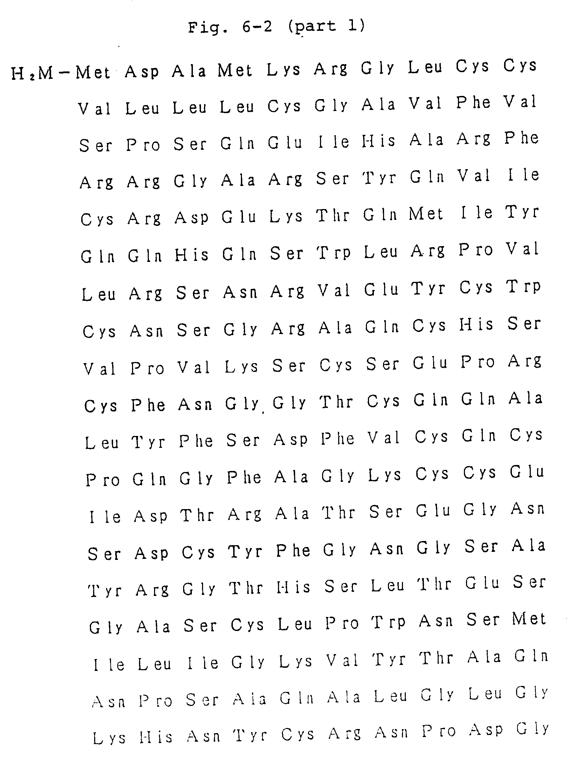

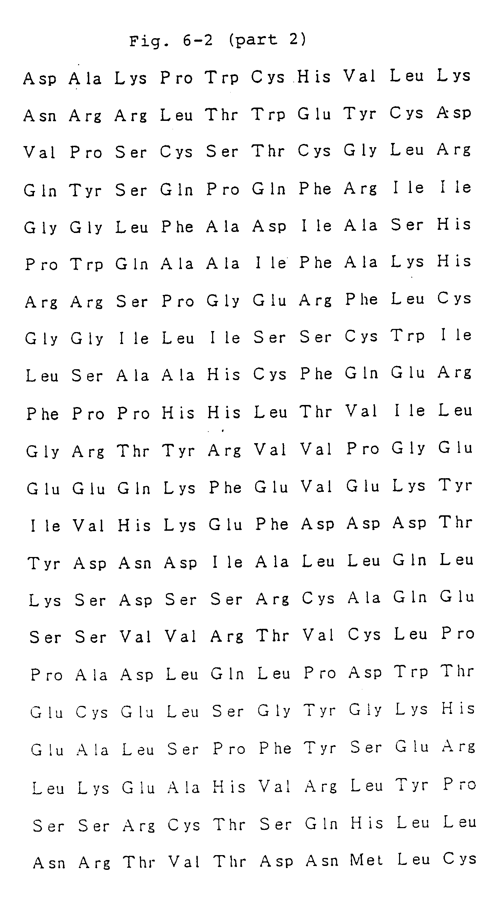

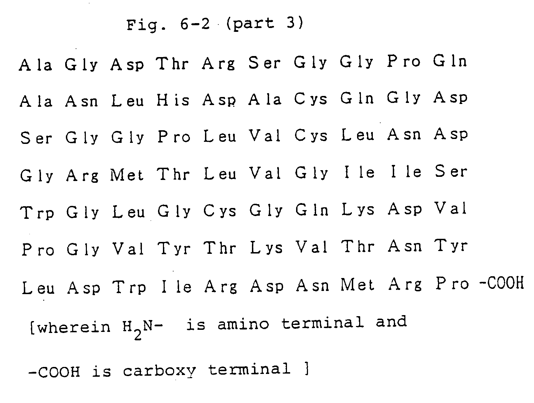

After ligating block IV (Bgl II-Eco RI fragment, about 480 base pairs) obtained in Example 2-1) with block V (Eco RI-Bal I fragment, about 1250 base pairs) obtained in Example 2-2) using the DNA ligation kit described above, the ligated product is subjected to ethanol precipitation. After drying under reduced pressure, the precipitate is cleaved with restriction enzyme Xho II in conventional manner. Next, 0.8% agarose gel electrophoresis is carried out to separate the Bgl II-Xho II fragment (about 1,500 base pairs, containing t-PA [II] gene). DNA is then isolated by electric elution. The entire base sequence of the thus constructed t-PA [II] gene is shown in Fig. 5. A deduced amino acid sequence is also shown in Fig. 6.

Example 3. Construction of t-PA (V) and improved t-PA (VI) gene

Constructions of the t-PAs (V) and (VI) gene are carried out based on the t-PA [II] gene, by referring to the following publications.

The objective genetic conversion is performed by the site-specific mutation induction method.

Publications:

Zoller, M.J. and Smith, M., Method in Enzymology, 100, 468-500 (1983); Zoller, M.J. and Smith, M., DNA, 3, 479-488 (1984); Morinaga, Y., et al., Bio/technology, 636-639 (July 1984); Adelman, J.P., et al., DNA, 2, 183-193 (1983); M13/pUC Sequence Manual [issued by Nippon Gene Science Room Co., Ltd.]

3-1) Construction of t-PA [V] gene

A) Design of M13mp9 (t-PA [II]) capable of inducing mutation

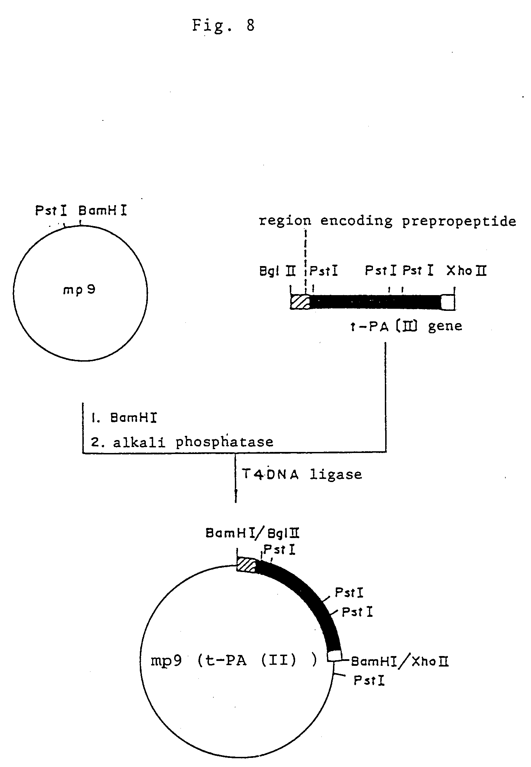

The t-PA [II] gene fragment described in detail in Example 2, 2-3) is ligated with double stranded M13mp9 DNA treated with restriction enzyme Bam HI and with alkaline phosphatase (manufactured by Takara Shuzo Co., Ltd.) using the DNA ligation kit described above. The ligation product described above is transfected in E. coli JM109 competent cells (manufactured by Takara Shuzo Co., Ltd.). Each clone of the resulting colorless phage plaque is infected to E. coli JM109. Single stranded DNA is isolated from the culture supernatant and double stranded (replicative) DNA is isolated from the E. coli cells according to the method of Messing [Messing, J., Methods in Enzymology, 101, 20-78 (1983)]. By pattern analysis of these double stranded DNAs after cleavage with restriction enzyme (Pst I) by agarose gel electrophoresis, clone, mp9 (t-PA [II]), in which t-PA [II] is inserted into mp9 DNA in the desired direction as shown in Fig. 8, is obtained.

That is, after cleaving a part of these DNAs with restriction enzyme Pst I, 0.8% agarose gel electrophoresis is carried out to give clone mp9 (t-PA [II]) showing a single band, respectively, at about 7,300 base pairs, about 840 base pairs, about 430 base pairs and about 80 base pairs. Single stranded DNA of this clone is used in the subsequent experiment for inducing the site-specific mutation.

## B) Synthesis of primer capable of inducing site-specific mutation

Synthetic oligonucleotide used for causing site-specific mutation of the improved t-PA [II] gene is synthesized by the $\beta$-cyanoethylphosphoamidide method using Model 380A DNA Synthesizer (manufactured by Applied Biosystems). Synthesis of DNA oligomer, removal of protection group, cleavage from resin and purification are carried out as instructed by the manual for Model 380A DNA Synthesizer. To induce mutation at the specific site, primer ① capable of inducing the following site-specific mutation and primer ② for sequencing by the dideoxy method using M13 phage vector [Carlson, J., et al., J. Biotechnology, 1, 253 (1984)] are prepared.

```
Amino acid sequence of          Gln Phe Arg Ile Lys Gly Gly Leu
         t-PA [II]:             Phe

DNA sequence of                  T CAG TTT CGC ATC AAA GGA GGG
         t-PA [II]:             CTC TTC GC
                                                      Ile
DNA sequence of                 5'T CAG TTT CGC ATC ATA GGA GGG
primer ① for                    CTC TTC GC  3'
inducing mutation:

DNA sequence of          5'  GCA GGC TGA CGT GGG AG   3'
primer ② for
sequencing:
```

The amino acid sequence of improved t-PA [II] and gene sequence are described in the foregoing two rows. The primer

① capable of inducing mutation is different in the underlined base from the gene sequence for improved t-PA [II].

## c) Induction of site-specific mutation

Hereafter shown is a way to construct a clone containing the base sequence of primer ① capable of inducing mutation, namely, improved t-PA [V] gene. After annealing mp9

(t-PA [II]) single stranded DNA described in Example 3, 3-1), A) and the primer ① capable of inducing mutation, the annealed product is converted into double stranded DNA, which is then transformed in E. coli JM109. Next, using the primer for sequencing, screening is carried out by DNA sequencing to isolate phage clone bearing the mutated t-PA [II] gene, namely, t-PA [V] gene. From this clone, double stranded (replicative) phage DNA is produced and this t-PA [V] gene is isolated.

## 5'-Terminal phosphorylation of synthetic oligomer

The DNA of primer ① for inducing site-specific mutation is phosphorylated by the procedure described in Example 2, 2-1).

## Production of heteroduplex DNA

Single stranded M13mp9 (t-PA [II]) DNA, 0.5 $\mu$g, and 1.5 $\mu$g of double stranded M13mp9 DNA cleaved with restriction enzyme Bam HI are heated in 30 $\mu$l of solution containing 2 pmoles of the phosphorylated primer ① for inducing mutation, 10 mM Tris-HCl (pH 7.5), 0.1 mM EDTA and 50 mM NaCl at 90°C (2 minutes), at 50°C (5 minutes), at 37°C (5 minutes) and at room temperature (10 minutes). To the solution is added 36 $\mu$l of a solution of 50 mM Tris-HCl (pH 8.0) containing 4 units of Klenow enzyme, 7 units of T4 DNA ligase, 0.1 mM EDTA, 12 mM $MgCl_2$, 10 mM dithiothreitol, 0.7 mM ATP, 0.07 mM dATP and 0.2 mM each of dGTP, dTTP and dCTP to initiate primer elongation. The mixture is reacted at 20°C for 2 hours and at 4°C for 15 hours.

Transformation was performed using the solution described above and E. coli JM109 competent cells (manufactured by Takara Shuzo Co., Ltd.) to form plaque. After picking up colorless plaque, phage is infected to E. coli JM109 to proliferate the same. Thereafter, template single stranded DNA is produced from the culture supernatant with respect to each clone. These single stranded DNAs are subjected only to Reaction "T" [Reactions "A" and "T" in Example 3-2] of the dideoxy method using primer ② for sequencing followed by polyacrylamide gel electrophoresis. After drying, the gel is analyzed by autoradiography. Based on the results, clone having the objective mutation sequence is identified. With respect to this clone, the culture supernatant described above is infected to E. coli JM109 and inoculated onto a plate to again perform isolation of a single plaque. Regarding the resulting clone of single plaque, single stranded DNA is produced as described above. Using these DNAs, DNA base sequence is firstly determined by the dideoxy method using primer ② for sequencing to obtain a clone mutated into the desired base sequence.

After this phage clone is infected to E. coli JM109 using the method of Messing as described in Example 2, double stranded DNA is produced. This double stranded DNA is cleaved with restriction enzyme Xho II, and 0.8% agarose gel electrophoresis is carried out to isolate a fragment of about 1500 base pairs containing t-PA [V] gene. Thereafter, DNA is recovered by electric elution.

Furthermore, the entire base sequence is determined by the dideoxy method, with respect to the thus produced DNA, whereby it is confirmed that the DNA is t-PA [V] gene. The entire base sequence of the thus constructed

t-PA [V] gene (but containing signal peptide -35 to -1) is shown in Fig. 5-2. The amino acid sequence deduced therefrom is also shown in Fig. 6-2.

3-2) Construction of improved t-PA [VI]

```
Amino acid sequence of        Gln Pro Gln Phe Arg Ile Lys Gly
         t-PA [II]:           Gly

DNA sequence of               GC CAG CCT CAG TTT CGC ATC AAA
         t-PA [II]:           GGA GGG C
                                                      Glu
DNA sequence of            5'GC CAG CCT CAG TTT GAA ATC AAA
primer ③ for                  GGA GGG C 3'
inducing mutation:
```

The procedures are similar to those described in Example 3, 3-1). Firstly, M13mp9 (t-PA [II]) is constructed for inducing mutation and primer for inducing site-specific mutation are then synthesized. The base sequence of the primer is described above; for construction of improved t-PA [VI] gene, 5'-end phosphorylated primer ③ for inducing mutation is used. Further after site-specific mutation induction, the entire base sequence is determined by the dideoxy method and it is confirmed that it has the desired base sequence. The improved t-PA [VI] is thus produced.

Next, this gene is integrated into vector pVYI according to the procedures described in Examples 4 and 5 in detail.

Example 4

Construction of vector pVYI

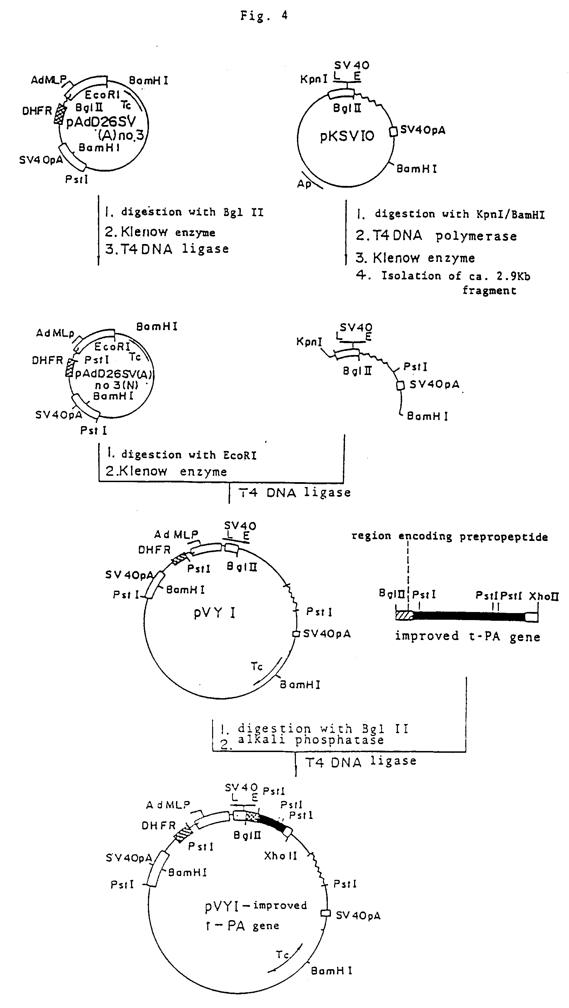

Vector pVYI is prepared as illustrated in Fig. 4.

A) Construction of pAd D26 SV(A) No. 3(N) and rendering the Eco RI cleavage site blunt ended

Firstly, pAd D26 SV(A) No. 3 [acquired from Dr. Hiroshi Handa in Tokyo University; known by the thesis in Mol. Cell. Biol., 2(11), (1982)] DNA is cleaved with restriction enzyme Bgl II (manufactured by Boehringer Mannheim-Yamanouchi Co., Ltd.) in conventional manner. Next, the DNA is rendered blunt ended in conventional manner using Klenow enzyme (manufactured by Boehringer Mannheim-Yamanouchi Co., Ltd.). After phenol treatment, enthanol precipitation and drying under reduced pressure, the precipitates are dissolved in distilled sterile water. After further performing ligation using the DNA ligation kit described above, E. coli HB101 competent cells (manufactured by Takara Shuzo Co., Ltd.) are transformed. Plasmid DNAs are acquired from transformants showing tetracycline resistance in conventional manner.

13

After cleaving a part of these DNAs with restriction enzyme Bgl II, 0.7% agarose gel electrophoresis is performed. As a result, clone bearing DNA that is not cleaved with Bgl II is obtained. After digesting [pAd D26 SV(A) No. 3(N) plasmid] DNA of this clone with restriction enzyme Eco RI in a conventional manner, the DNA is made blunt ended with Klenow enzyme as described above. After phenol treatment, enthanol precipitation and drying under reduced pressure, the precipitates are dissolved in distilled sterile water.

B) Isolation of Kpn I-Bam HI (about 2,900 base pairs) from pKSV 10 and rendering blunt ended

After cleaving pKSV 10 (manufactured by Pharmacia Fine Chemicals) DNA with restriction enzymes Kpn I and Bam HI in conventional manner, the DNA is rendered blunt ended using T4 DNA polymerase (manufactured by Takara Shuzo Co., Ltd.) and Klenow enzyme (the Laboratory Manual, pages 114-121). Next, 0.7% agarose gel electrophoresis is performed to separate a fragment of about 2,900 base pairs. Then the fragment is subjected to electric elution to recover the DNA.

C) Construction of pVYI

After ligating the DNA fragment obtained in A) and the DNA fragment obtained in B) using the DNA ligation kit, E. coli HB101 competent cells (described above) are transformed with the ligated product.

From the transformants showing tetracycline resistance, plasmid DNAs are prepared in conventional manner. After cleaving a part of these plasmid DNAs with restriction enzyme Pst I (manufactured by Boehringer Mannheim-Yamanouchi Co., Ltd.) in conventional manner, 1.0% agarose gel electrophoresis is carried out. As a result, a clone (plasmid pVYI) showing bands of about 3,400 base pairs, about 3,200 base pairs and about 1,400 base pairs is obtained. This clone E. coli HB101/pVYI has been deposited in the Fermentation Research Institute of the Agency of Industrual Science & Technology of Japan under Registration No. P-9625 (FERM BP 2106).

Example 5. Integration of t-PA (V) and (VI) genes into vector pVYI

After plasmid pVYI DNA prepared in Example 4) is cleaved with restriction enzyme Bgl II in conventional manner, dephosphorylation is performed using alkaline phosphatase (manufactured by Takara Shuzo Co., Ltd.) and treatment with phenol 3 times, ethanol precipiation and drying under reduced pressure follow. The residue is then dissolved in sterile distilled water.

After this DNA is ligated with the Bgl II-Xho II fragment (about 1,500 base pairs) prepared in Examples 2, 2-3) using the DNA ligation kit, the ligation product is transformed on the aforesaid E. coli HB101 competent cells. Plasmid DNAs are acquired from transformants showing tetracycline resistance in conventional manner. After cleaving these DNAs with restriction enzymes (Bgl II, Pst I), agarose gel electrophoresis is performed. By analysis of the agarose gel electrophoresis pattern, clones in which the t-PA [V] gene is incorporated into vector pVYI in the desired direction are selected. Firstly, after cleaving a part of these DNAs with restriction enzyme Bgl II, electrophoresis is carried out in a concentration of 0.8% agarose gel to give clones showing a band of the fragment of about 1,500 base pairs. When the Bgl II-Xho II fragment is bound to the Bgl II fragment of pVYI, the bound Xho II and Bgl II portion can be cleaved by restriction enzyme Bgl II due to the aforesaid isoshizomer configuration.

After further cleaving a part of plasmid DNAs of these clones with restriction enzyme Pst I, electrophoresis is carried out in a concentration of 0.8% agarose gel to give a clone showing one band at about 3,400 base pairs, one at about 2,300 base pairs, 2 bands at about 1,400 base pairs, one band at about 800 base pairs and one band at about 80 base pairs. Using the clone (plasmid pVYI-t-PA [V]), plasmid DNA is produced in large quantities based on the Laboratory Manual at page 86 described above.

In a similar manner, improved t-PA [VI] gene is integrated into vector pVYI.

Example 6. Expression of improved t-PA in CHO cells

Plasmid pVYI-improved t-PA [VI] gene is transfected in DHFR-deficient CHO cells [Urlaub, et al., Proc. Natl. Acad. Sci. USA, 77(7), 4216-4220 (1980)] by the calcium phosphate method [Graham, et al., Virology, 52, 456 (1973)]. It is found that the transformant clone obtained from selection medium [MEM ALPHA (-), GIBCO] in the presence of methotrexate (MTX) produces t-PA activity of 50 to 100 U/ml (value determined by fibrin/agarose plate method, later described). This clone is used for subsequent studies. As production medium, GIT medium (manufactured by Wako Pure Chemical Industry Co., Ltd.) is used and 20 KIU/ml [SIGMA] of aprotinin is supplemented.

Example 7. Purification of improved t-PA from the culture supernatant of CHO cells

The culture supernatant obtained in Example 6 is partially purified through anti-t-PA monoclonal antibody affinity column. Monoclonal antibody-producing hybridoma to human melanoma cell-derived t-PA is produced in conventional manner. The antibody-producing hybridoma is inoculated to mice and monoclonal antibody (subclass: IgGI) developed in the ascites is recovered and purified using Protein A Cellulofine (manufactured by Biochemical Industry Co., Ltd.) and MAPS buffer system for purifying monoclonal antibody manufactured by Biorad Laboratories. The antibody is coupled to CNBr-activated Sepharose (manufactured by Pharmacia Fine Chemicals) in a ratio Of 4 mg per 1 ml of the gel in conventional manner.

The antibody gel, 24 ml, is mixed with 4 liters of the culture supernatant. After gently shaking overnight at 4°C, the gel is packed in a column (diameter of 1.5 cm x 20 cm). Next, the gel is sequentially washed with 125 ml each of ① Tris-hydrochloride buffer, pH 7.4 (buffer A) containing 25 KIU/ml of aprotinin (manufactured by SIGMA) and 0.01% (w/v) of Tween 80, ② buffer A containing 0.5 M NaCl, ③ buffer A containing 4 M of urea and ④ buffer A. The improved t-PA bound to the gel is eluted with 0.2 M glycine-hydrochloride buffer, pH 2.5 (containing 25 KIU/ml of aprotinin and 0.01% (w/v) of Tween 80). The active fractions are recovered and combined. After dialyzing to 10 mM Tris-hydrochloride buffer, pH 7.4 (containing 25 KIU/ml of aprotinin and 0.01% (w/v) of Tween 80) overnight, the dialysate is concentrated to 20- to 30-fold with a vacuum centrifuging concentrater (Speed VAC, manufactured by SAVANT Inc.). The concentrate is again dialyzed to 10 mM Tris-hydrochloride buffer, pH 7.4 (contaianing 0.15 M NaCl, 25 KIU/ml of aprotinin and 0.01% (w/v) of Tween 80) overnight and used for subsequent evaluation in vitro and in vivo. Finally, the specific activity is increased by 3,700 to 5,000 times and 36 to 42% t-PA activity (determined by fibrin/agarose plate method) is recovered.

This active fraction is analyzed by SDS-electrophoresis and silver staining. Under the reducing conditions, a very strong band is noted around 54 kilodaltons, together with several other bands. Furthermore the gel after the electrophoresis is treated with 2.5% (w/v) Triton X-100 and then put on fibrin/agarose plate to perform fibrin autography at 37°C, whereby a dissolved band is noted at about 50 kilodaltons. On the same plate, naturally occurring t-PA appears at about 60 kilodaltons. The results strongly suggest that t-PA adsorbed to the antibody affinity column and eluted by the procedure would be the improved t-PA having a molecular weight theoretically smaller by 10,000 than that of naturally occurring type.

Example 8. Measurement of specific activity of improved t-PA

The protein amount of the partially purified improved t-PA is determined by measuring the total protein according to the method of Bradford [Bradford, Anal. Biochem., 72, 248 (1976)] using bovine serum albumin as standard protein. The amount of t-PA antigen is measured by ELISA. ELISA is sandwich type using the monoclonal antibody used for the antibody column described above and biotinated rabbit anti-t-PA antibody (manufactured by American Diagnostica Inc.) and a color is formed using biotinated horse radish peroxidase streptavidin complex (manufactured by Amersham Co., Ltd.) and its substrate (3,3',5,5'-tetramethylbentidine). As standard t-PA, human melanoma cell-derived single stranded t-PA manufactured by American Diagnostica Inc. is used.

Fibrinolytic activity is determined by the fibrin/agarose plate method and the $^{125}$I-labeled fibrin film dissolution method. The fibrin/agarose plate is prepared by adding agar to 95% coagulated fibrinogen.

The $^{125}$I-labeled fibrin film dissolution method is performed according to the method of Hoyraerts et al. [J. Biol. Chem., 257, 2912 (1982)]. That is, a suitable amount of $^{125}$I-labeled fibrinogen (manufactured by ICN Biomedical Co., Ltd.) is added to 1.8 μM fibrinogen and the mixture is charged in a 96 well microtiter plate (manufactured by Limbro Co., Ltd.) - 50 μl per well - followed by drying at 40°C overnight. Then 100 μl per well of 1.6 U/ml of thrombin (manufactured by Mochida Pharmaceutical Co., Ltd.) is added. The mixture is allowed to stand at 37°C for 4 hours to fibrinate. After washing twice with 10 mM phosphate buffer containing 0.2% calf serum albumin and 0.9% NaCl, the plate is provided for determination of the activity. In each well 50 μl of 200 nM plasminogen is charged and further 50 μl of t-PA standard or improved t-PA is added thereto. After mixing them, the mixture is reacted at 37°C for 2 hours. From each well 50 μl is taken and the dissolved $^{125}$I-fibrin is measured with Auto Well Gamma Counter manufactured by Aloka Inc. and by comparing with the standard curve prepared using standard t-PA, the fibrinolytic activity of improved t-PA is calculated. The standard t-PA used is human melanoma cell-derived t-PA manufactured by Bioscott Inc. standardized according to International t-PA Standard [Gaffuey and Curtis, Thromb. Haemostas., 53, 134 (1985)].

15

The specific activity value calculated from the activity value determined by the $^{125}$I-fibrin film method and the antigen amount determined by ELISA is 300,000 to 420,000 U/mg antigen.

Example 9. Affinity of improved t-PA to fibrin and activation with fibrin

According to Verheijen, et al. [[EMBO J., _5_, 3525 (1986)], affinity of improved t-PA to fibrin is examined. To fibrinogen in various concentrations, improved or naturally occurring t-PA (1,000 U/ml) is added and 1 U of thrombin is further added thereto followed by reacting at room temperature for 3 minutes. The formed fibrin clot is precipitated by centrifugation at 16,000 r.p.m. for 8 mins. and the amount of t-PA which is not bound to fibrin in the supernatant is determined by activity measurement by the fibrin/agarose plate method;

improved t-PA [VI] shows an equal affinity to fibrin, as compared to natural type. In order to examine the plasminogen activation rate of the improved t-PA in the presence or absence of fibrin, the following run is carried out. Using a 96 well microtiter plate, naturally occurring or improved t-PA is added to 0.1 M Tris-hydrochloride buffer, pH 7.5, containing 0.3 mM synthetic p-nitroanilide-tripeptide synthetic substrate S-2251 (H-D-Val-Leu-Lys-pNA.HCl, manufactured by Kabi Inc.), 0.13 $\mu$M plasmin-free plasminogen, 120 $\mu$g/ml of DESAFIB™ (manufactured by American Diagnostica Inc.) and 0.1% Tween 80 to make the whole volume 200 $\mu$l. The system is kept at 37°C. After a definite time period, absorbancy (A 405 nm) at wavelength of 405 nm is measured with Titertech Multiscan Model 310.

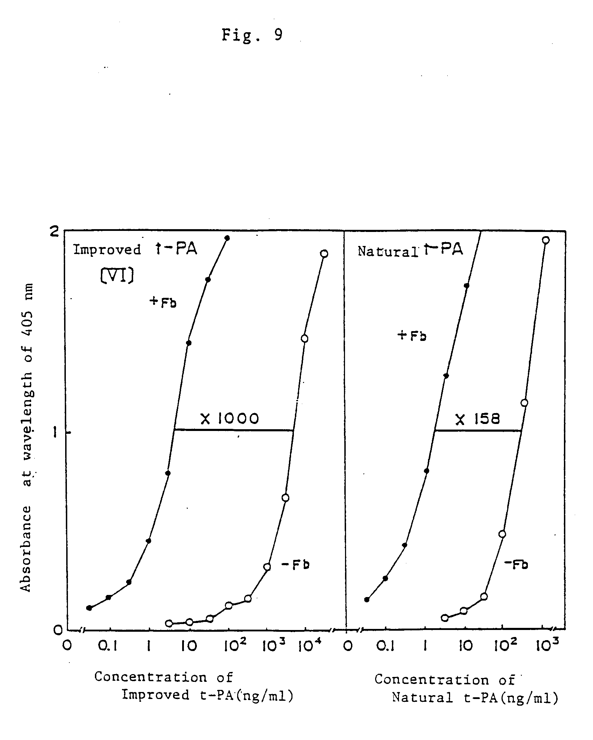

Th dose-response curve of improved t-PA [VI] and naturally occurring t-PA in amidolytic activity is shown in Fig. 9. When the activation rate is compared by shift of the dose-response curve due to the addition of DESAFIB™, naturally occurring t-PA is 158 times whereas improved t-PA [VI] reaches 1000 times. This is due to the fact that the activity of improved t-PA [VI] in the absence of DESAFIB™ is lower by about 1/20 than that of natural t-PA.

Example 10. Analysis of improved t-PA on fibrinolytic activity in rabbit blood flow

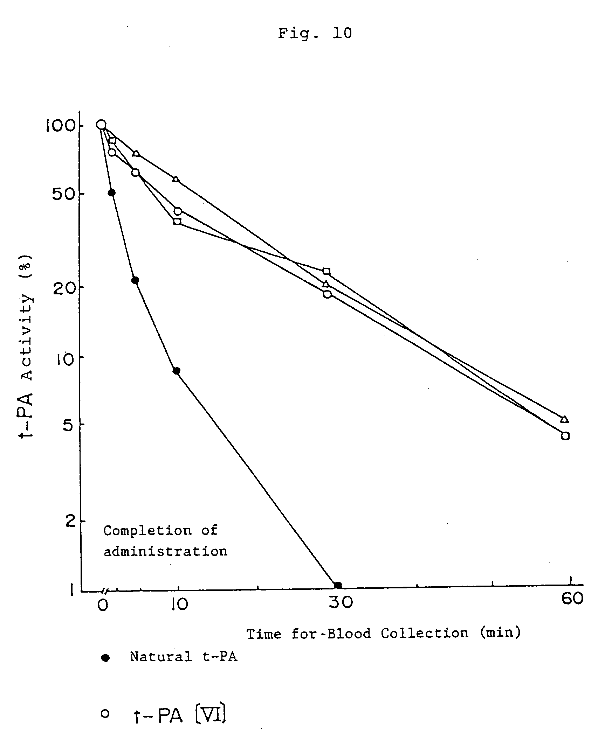

Pharmacokinetic is examined in view of the activities of naturally occurring t-PA (n-t-PA) and the improved t-PA of the present invention in rabbit. As is clear from Fig. 10, the improved t-PA shows remarkable prolongation of the half life in the activity value. More specifically, natural t-PA shows the half life for 1 to 2 minutes, whereas that of the improved t-PA shows 8 to 15 minutes. In addition, it is noted that the activity value of 5% (the value 30 seconds after the administration is made 100%) still remains in the improved t-PA even 60 minutes after the administration. On the other hand, natural t-PA shows the activity value of 0.1% 60 minutes after.

This experiment is carried out as follows.

For the run, Japanese white rabbit weighing 2.4 kg is used. Under pentobarbital anesthesia, t-PA is administered through the peripheral vein of the ear. The dose is 15,400 U/0.8 ml/rabbit of improved t-PA and 5,400 U/0.8 ml/rabbit of n-t-PA (value determined by the fibrin plate method). Subsequently, 2.5 ml each of blood is collected from the femoral artery using a catheter in various time intervals (0.5 to 60 minutes) and taken in 1/9 volume of sodium citrate (3.8%). Within 30 minutes after blood collection, centrifugation is performed at a low speed to separate plasma. Using the separated plasma, the t-PA activity in blood is measured.

(1) Measurement of t-PA activity

After diluting 0.2 ml of plasma 16-fold with 3 mM glacial acetic acid, the dilution is centrifuged at a low speed to give precipitates. The precipitates are dissolved in 20 mM Tris-HCl, pH 7.4-140 mM NaCl buffer of a volume equivalent to the plasma to obtain euglobulin fraction. The t-PA activity is determined by adding this euglobulin fraction into fibrin/agarose plate. After incubation of the plate at 37°C for 16 hours, the t-PA activity is observed as plaque. The fibrin/agarose plate is prepared as follows.

Commercially available fibrinogen (fraction I of corn) is used for preparation of fibrin/agarose plate as plasminogen-rich fibrinogen. The final concentration of plasminogen-rich fibrinogen is 1.5 mg/ml in 20 mM Tris-HCl buffer, pH 7.4, containing 130 mM NaCl and $10^{-4}$M CaCl$_2$. The final agarose concentration is 0.75% in the same buffer. Thrombin (40 NIH units/ml), 100 $\mu$l, is added to 10 ml of fibrinogen agarose solution to prepare a plate. The standard curve for the fibrin/agarose plate method is obtained by diluting t-PA used for administration to the animal to 0.1 to 10,000 U/ml. The thus determined t-PA activity in blood is expressed by per cent, using the t-PA activity obtained by collecting blood 30 seconds after the administration as 100%.

16

Example 11. Stability of improved t-PA [VI] to heat and acids

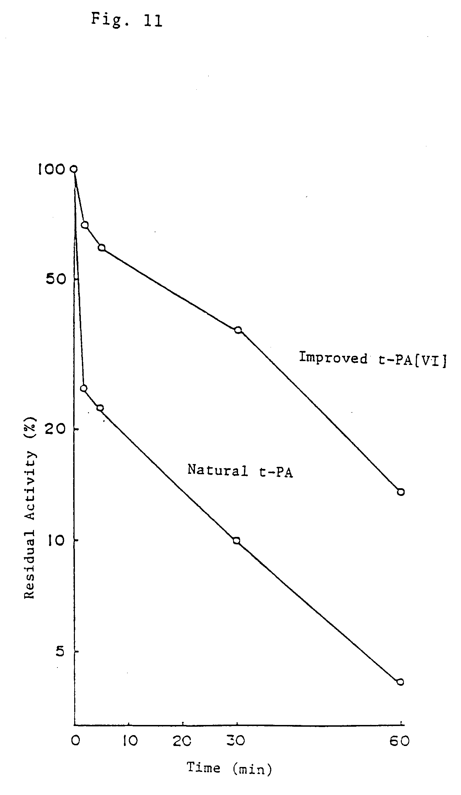

Stability of improved t-PA [VI] is examined. With respect to stability to heat, improved t-PA [VI] and natural t-PA are diluted with 50 mM Tris buffer (containing 100 mM NaCl and 0.01% Tween 80), pH 7.4, to a concentration of 100 $\mu$g/ml, respectively. Each solution is allowed to stand in boiling water (98°C) for 2 to 60 minutes. After cooling, the remaining activity is determined by the fibrin plate method. As shown in Fig. 11, reduction in the activity of improved t-PA [VI] is very gentle as compared to that of natural t-PA. For example after heat treatment for 2 minutes, the activity of natural t-PA is reduced to 25%, whereas in improved t-PA [VI] activity of 71% still remains.

Improved t-PA [VI] and natural t-PA are dissolved in 0.5 N hydrochloric acid in a concentration of 100 $\mu$g/ml followed by allowing to stand at room temperature for 30 minutes. After neutralization, the activity is determined by the fibrin plate method. In the improved t-PA, activity changes not at all, whereas in natural t-PA, the activity is reduced by 50%.

Example 12 Inhibition of LAF activity with improved t-PA [VI]

Improved t-PA [VI] and natural t-PA are suitably diluted with tissue culture medium RPMI 1640 containing 7% calf fetal serum and 58 $\mu$M 2-mercaptoethanol. This dilution, 100 $\mu$l, is charged to a 96 well flat bottomed multiplate and 50 $\mu$l each of cell suspension containing thymocyte ($2 \times 10^7$ cells/ml) collected from male C3H/HeJ mice of 4 to 6 weeks age and Concanavalin A (1.2 $\mu$g/ml) and 50 $\mu$l of IL-I (4 units/ml, Genzyme Inc.) are added followed by culturing for 48 hours in an incubator at 37°C containing 5% carbon dioxide. Then, $^3$H-thymidine is added (0.5 $\mu$Ci/20 $\mu$l/well). After culturing for a further 18 hours, the cells are gathered on a glass fiber filter using a cell harvester and the amount of $^3$H-thymidine incorporated into the cells is measured with a liquid scintillation counter thereby to determine the LAF activity.

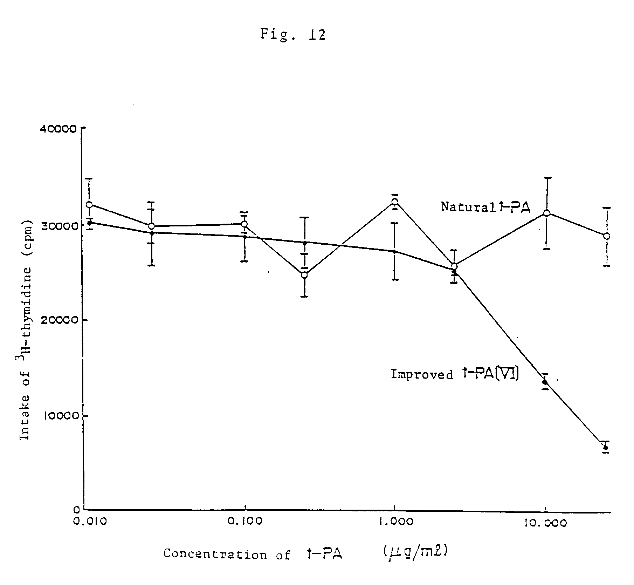

As shown in Fig. 12, natural t-PA does not inhibit the LAF activity at all but the improved t-PA significantly inhibits the LAF activity by IL-1. When tested with the solvent alone, no influence is noted.

Example 13. Antiinflammatory activity based on the action on denatured protein

1) Preparation of denatured protein

After a protein solution (5 mg/ml) is incubated in 0.1 N HCl or 0.1 N NaOH at 37°C for 2 to 3 hours, the protein solution is neutralized with the same amount of NaOH or HCl to prepare the denatured protein.

2) Affinity of improved t-PA [VI] to denatured protein

Method:

According to the procedure shown below, denatured protein is adhered to a piece of nitrocellulose film. Next, the amount of improved t-PA bound to the piece of nitrocellulose film is measured, whereby the affinity of the improved t-PA to denatured protein is determined.

A piece of nitrocellulose film is charged
into 20 mM Tris-hydrochloride buffer, pH
7.5 (TBS buffer) containing 140 mM NaCl

drying

Denatured protein (50 µg/10 µl) is
dropped onto the piece of nitrocellulose
film

drying

Blocking with 3% gelatin solution

washing

The piece of nitrocellulose film is
charged in 1 µg of improved t-PA/ml.

washing

Plasminogen and synthetic substrate
S-2251 are added followed by incubation
at 37°C (quantitative assay for the
improved t-PA adsorbed)

Measurement of OD 405

Results:

As shown in the following table, the improved t-PA shows affinity to IgG treated with HCl and to the albumin treated with HCl and to the albumin treated with NaOH. On the other hand, the improved t-PA does not show affinity to intact IgG and albumin.

EP 0 386 240 B1

| Kind of protein adhered to cellulose film | Amount of improved t-PA bound (ng/piece of cellulose film) |
|---|---|
| Buffer | 0 |
| IgG | 0.2 |
| HCl-treated IgG | 1.35 |
| Albumin | 0 |
| NaOH-treated albumin | 0.9 |

3) Activation of improved t-PA [VI] with denatured protein

Method:

Plasminogen (Kabi Inc.), 0.0078 CU/10 $\mu$l, 100 $\mu$l of 3 mM synthetic substrate S-2251 and various amounts of TBS buffer are added to the reaction solution of improved t-PA activator (denatured protein or BrCN-treated fibrinogen or the like) in various concentrations to make the amount of reaction solution 0.275 ml. The improved t-PA, 2.5 ng/25 $\mu$l, is added to the reaction solution to initiate the reaction. After reacting for a definite time period, 2% SDS solution is added in an equimolar amount to the reaction solution to discontinue the reaction. By measuring OD 405, the activity of improved t-PA is determined.

Results:

As shown in Fig. 13, NaOH-treated albumin and HCl-treated IgG exhibit potent activation of the improved t-PA. Particularly in the HCl-treated IgG, the activation is strong and the activity of the HCl-treated IgG is substantially identical with that of the BrCN-treated fibrinogen in a concentration smaller by one-several tenths time than in the BrCN-treated fibrinogen. However, the intact albumin and IgG show no activation.

4) Degradation of denatured protein with improved t-PA [VI]

Method:

After reacting the denatured protein with the improved t-PA under the same conditions as the method described in "3) Activation of improved t-PA with denatured protein" except for adding no synthetic substrate S-2251 to the reaction system and fixing the amount of denatured protein to 133 $\mu$g/ml, SDS gel electrophoresis is performed in the presence of $\beta$-mercaptoethanol.

Results:

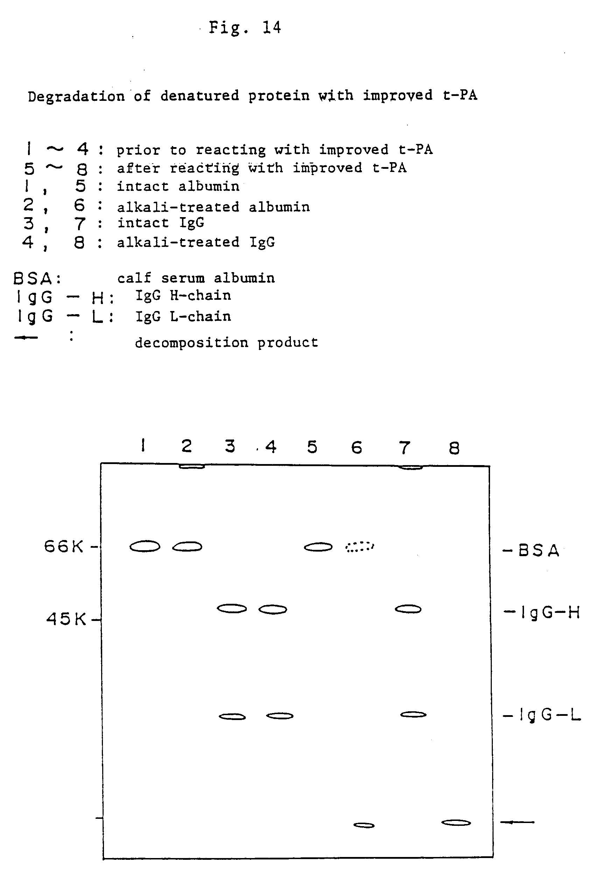

As shown in Fig. 14, the protein denatured by NaOH treatment or HCl treatment causes disappearance of bands and formation of degradation products, indicating decomposition of the denatured protein. On the other hand, with the intact albumin, no change is noted in band even after reacting with the improved t-PA and therefore no degradation of the denatured protein is noted.

**Claims**

1. An improved tissue plasminogen activator having the following amino acid sequence in which, compared to human t-PA, kringle 1 is deleted and Arg 275 is substituted by Glu :

19

```
H₂N ─ Ser Tyr Gln Val Ile Cys Arg Asp Glu Lys
      Thr Gln Met Ile Tyr Gln Gln His Gln Ser
      Trp Leu Arg Pro Val Leu Arg Ser Asn Arg
      Val Glu Tyr Cys Trp Cys Asn Ser Gly Arg
      Ala Gln Cys His Ser Val Pro Val Lys Ser
      Cys Ser Glu Pro Arg Cys Phe Asn Gly Gly
      Thr Cys Gln Gln Ala Leu Tyr Phe Ser Asp
      Phe Val Cys Gln Cys Pro Glu Gly Phe Ala
      Gly Lys Cys Cys Glu Ile Asp Thr Arg Ala
      Thr Ser Glu Gly Asn Ser Asp Cys Tyr Phe
      Gly Asn Gly Ser Ala Tyr Arg Gly Thr His
      Ser Leu Thr Glu Ser Gly Ala Ser Cys Leu
      Pro Trp Asn Ser Met Ile Leu Ile Gly Lys
      Val Tyr Thr Ala Gln Asn Pro Ser Ala Gln
      Ala Leu Gly Leu Gly Lys His Asn Tyr Cys
      Arg Asn Pro Asp Gly Asp Ala Lys Pro Trp
      Cys His Val Leu Lys Asn Arg Arg Leu Thr
      Trp Glu Tyr Cys Asp Val Pro Ser Cys Ser
      Thr Cys Gly Leu Arg Gln Tyr Ser Gln Pro
      Gln Phe  Y  Ile Lys Gly Gly Leu Phe Ala
      Asp Ile Ala Ser His Pro Trp Gln Ala Ala
      Ile Phe Ala Lys His Arg Arg Ser Pro Gly
      Glu Arg Phe Leu Cys Gly Gly Ile Leu Ile
      Ser Ser Cys Trp Ile Leu Ser Ala Ala His
      Cys Phe Gln Glu Arg Phe Pro Pro His His
      Leu Thr Val Ile Leu Gly Arg Thr Tyr Arg
      Val Val Pro Gly Glu Glu Glu Gln Lys Phe
      Glu Val Glu Lys Tyr Ile Val His Lys Glu
```

```
Phe Asp Asp Asp Thr Tyr Asp Asn Asp Ile
Ala Leu Leu Gln Leu Lys Ser Asp Ser Ser
Arg Cys Ala Gln Glu Ser Ser Val Val Arg
Thr Val Cys Leu Pro Pro Ala Asp Leu Gln
Leu Pro Asp Trp Thr Glu Cys Glu Leu Ser
Gly Tyr Gly Lys His Glu Ala Leu Ser Pro
Phe Tyr Ser Glu Arg Leu Lys Glu Ala His
Val Arg Leu Tyr Pro Ser Ser Arg Cys Thr
Ser Gln His Leu Leu Asn Arg Thr Val Thr
Asp Asn Met Leu Cys Ala Gly Asp Thr Arg
Ser Gly Gly Pro Gln Ala Asn Leu His Asp
Ala Cys Gln Gly Asp Ser Gly Gly Pro Leu
Val Cys Leu Asn Asp Gly Arg Met Thr Leu
Val Gly Ile Ile Ser Trp Gly Leu Gly Cys
Gly Gln Lys Asp Val Pro Gly Val Tyr Thr
Lys Val Thr Asn Tyr Leu Asp Trp Ile Arg
Asp Asn Met Arg Pro-COOH
```

wherein Y represents Glu, $H_2N$- represents the amino terminal, and -COOH represents the carboxy terminal.

2. An improved tissue plasminogen activator gene coding for the amino acid sequence defined in claim 1.

3. A composition for treatment of thrombosis comprising as an active ingredient an improved tissue plasminogen activator according to claim 1.

4. A process for preparing improved tissue plasminogen activator according to claim 1, which comprises expressing DNA encoding said improved tissue plasminogen activator in a suitable host and recovering the expressed improved tissue plasminogen activator.

5. A process according to claim 4, wherein the host is CHO cells.

**Patentansprüche**

1. Verbesserter Gewebe-Plasminogen-Aktivator mit der folgenden Aminosäuresequenz, in der im Vergleich zu humanem t-PA der Kringel 1 deletiert und Arg 275 durch Glu substituiert ist:

```
H₂N — Ser Tyr Gln Val Ile Cys Arg Asp Glu Lys
      Thr Gln Met Ile Tyr Gln Gln His Gln Ser
      Trp Leu Arg Pro Val Leu Arg Ser Asn Arg
      Val Glu Tyr Cys Trp Cys Asn Ser Gly Arg
      Ala Gln Cys His Ser Val Pro Val Lys Ser
      Cys Ser Glu Pro Arg Cys Phe Asn Gly Gly
      Thr Cys Gln Gln Ala Leu Tyr Phe Ser Asp
      Phe Val Cys Gln Cys Pro Glu Gly Phe Ala
      Gly Lys Cys Cys Glu Ile Asp Thr Arg Ala
      Thr Ser Glu Gly Asn Ser Asp Cys Tyr Phe
      Gly Asn Gly Ser Ala Tyr Arg Gly Thr His
      Ser Leu Thr Glu Ser Gly Ala Ser Cys Leu
      Pro Trp Asn Ser Met Ile Leu Ile Gly Lys
      Val Tyr Thr Ala Gln Asn Pro Ser Ala Gln
      Ala Leu Gly Leu Gly Lys His Asn Tyr Cys
      Arg Asn Pro Asp Gly Asp Ala Lys Pro Trp
      Cys His Val Leu Lys Asn Arg Arg Leu Thr
      Trp Glu Tyr Cys Asp Val Pro Ser Cys Ser
      Thr Cys Gly Leu Arg Gln Tyr Ser Gln Pro
      Gln Phe  Y  Ile Lys Gly Gly Leu Phe Ala
      Asp Ile Ala Ser His Pro Trp Gln Ala Ala
      Ile Phe Ala Lys His Arg Arg Ser Pro Gly
      Glu Arg Phe Leu Cys Gly Gly Ile Leu Ile
      Ser Ser Cys Trp Ile Leu Ser Ala Ala His
      Cys Phe Gln Glu Arg Phe Pro Pro His His
      Leu Thr Val Ile Leu Gly Arg Thr Tyr Arg
      Val Val Pro Gly Glu Glu Glu Gln Lys Phe
      Glu Val Glu Lys Tyr Ile Val His Lys Glu
```

```
Phe Asp Asp Asp Thr Tyr Asp Asn Asp Ile
Ala Leu Leu Gln Leu Lys Ser Asp Ser Ser
Arg Cys Ala Gln Glu Ser Ser Val Val Arg
Thr Val Cys Leu Pro Pro Ala Asp Leu Gln
Leu Pro Asp Trp Thr Glu Cys Glu Leu Ser
Gly Tyr Gly Lys His Glu Ala Leu Ser Pro
Phe Tyr Ser Glu Arg Leu Lys Glu Ala His
Val Arg Leu Tyr Pro Ser Ser Arg Cys Thr
Ser Gln His Leu Leu Asn Arg Thr Val Thr
Asp Asn Met Leu Cys Ala Gly Asp Thr Arg
Ser Gly Gly Pro Gln Ala Asn Leu His Asp
Ala Cys Gln Gly Asp Ser Gly Gly Pro Leu
Val Cys Leu Asn Asp Gly Arg Met Thr Leu
Val Gly Ile Ile Ser Trp Gly Leu Gly Cys
Gly Gln Lys Asp Val Pro Gly Val Tyr Thr
Lys Val Thr Asn Tyr Leu Asp Trp Ile Arg
Asp Asn Met Arg Pro-COOH
```

in der Y Glu darstellt, $H_2N-$ den Aminoterminus und -COOH den Carboxyterminus darstellt.

2. Verbessertes Gewebe-Plasminogen-Aktivator-Gen, das für die in Anspruch 1 definierte Aminosäuresequenz kodiert.

3. Zusammensetzung zur Behandlung von Thrombosen, umfassend als aktiven Inhaltsstoff einen verbesserten Gewebe-Plasminogen-Aktivator nach Anspruch 1.

4. Verfahren zum Herstellen des verbesserten Gewebe-Plasminogen-Aktivators nach Anspruch 1, das umfaßt, DNS, die für den verbesserten Gewebe-Plasminogen-Aktivator kodiert, in einem geeigneten Wirt zu exprimieren und den exprimierten verbesserten Gewebe-Plasminogen-Aktivator wiederzugewinnen.

5. Verfahren nach Anspruch 4, bei dem der Wirt CHO-Zellen sind.

**Revendications**

1. Un activateur plasminogène du tissu amélioré ayant la séquence d'amino-acide suivante dans laquelle, comparé au t-PA humain, le kringle 1 est enlevé et l'Arg 275 est remplacé par Glu :

```
H₂N — Ser Tyr Gln Val Ile Cys Arg Asp Glu Lys
      Thr Gln Met Ile Tyr Gln Gln His Gln Ser
      Trp Leu Arg Pro Val Leu Arg Ser Asn Arg
      Val Glu Tyr Cys Trp Cys Asn Ser Gly Arg
      Ala Gln Cys His Ser Val Pro Val Lys Ser
      Cys Ser Glu Pro Arg Cys Phe Asn Gly Gly
      Thr Cys Gln Gln Ala Leu Tyr Phe Ser Asp
      Phe Val Cys Gln Cys Pro Glu Gly Phe Ala
      Gly Lys Cys Cys Glu Ile Asp Thr Arg Ala
      Thr Ser Glu Gly Asn Ser Asp Cys Tyr Phe
      Gly Asn Gly Ser Ala Tyr Arg Gly Thr His
      Ser Leu Thr Glu Ser Gly Ala Ser Cys Leu
      Pro Trp Asn Ser Met Ile Leu Ile Gly Lys
      Val Tyr Thr Ala Gln Asn Pro Ser Ala Gln
      Ala Leu Gly Leu Gly Lys His Asn Tyr Cys
      Arg Asn Pro Asp Gly Asp Ala Lys Pro Trp
      Cys His Val Leu Lys Asn Arg Arg Leu Thr
      Trp Glu Tyr Cys Asp Val Pro Ser Cys Ser
      Thr Cys Gly Leu Arg Gln Tyr Ser Gln Pro
      Gln Phe  Y  Ile Lys Gly Gly Leu Phe Ala
      Asp Ile Ala Ser His Pro Trp Gln Ala Ala
      Ile Phe Ala Lys His Arg Arg Ser Pro Gly
      Glu Arg Phe Leu Cys Gly Gly Ile Leu Ile
      Ser Ser Cys Trp Ile Leu Ser Ala Ala His
      Cys Phe Gln Glu Arg Phe Pro Pro His His
      Leu Thr Val Ile Leu Gly Arg Thr Tyr Arg
      Val Val Pro Gly Glu Glu Glu Gln Lys Phe
    _ Glu Val Glu Lys Tyr Ile Val His Lys Glu
```

24

```
Phe Asp Asp Asp Thr Tyr Asp Asn Asp Ile
Ala Leu Leu Gln Leu Lys Ser Asp Ser Ser
Arg Cys Ala Gln Glu Ser Ser Val Val Arg
Thr Val Cys Leu Pro Pro Ala Asp Leu Gln
Leu Pro Asp Trp Thr Glu Cys Glu Leu Ser
Gly Tyr Gly Lys His Glu Ala Leu Ser Pro
Phe Tyr Ser Glu Arg Leu Lys Glu Ala His
Val Arg Leu Tyr Pro Ser Ser Arg Cys Thr
Ser Gln His Leu Leu Asn Arg Thr Val Thr
Asp Asn Met Leu Cys Ala Gly Asp Thr Arg
Ser Gly Gly Pro Gln Ala Asn Leu His Asp
Ala Cys Gln Gly Asp Ser Gly Gly Pro Leu
Val Cys Leu Asn Asp Gly Arg Met Thr Leu
Val Gly Ile Ile Ser Trp Gly Leu Gly Cys
Gly Gln Lys Asp Val Pro Gly Val Tyr Thr
Lys Val Thr Asn Tyr Leu Asp Trp Ile Arg
Asp Asn Met Arg Pro-COOH
```

où Y représente Glu, $H_2N-$ représente le groupe amino terminal et -COOH représente le groupe carboxylique terminal.

2. Un codage de gène d'activateur plasminogène du tissu amélioré pour la séquence d'amino-acide définie dans la revendication 1.

3. Une composition pour le traitement de la thrombose comprenant en tant qu'ingrédient actif un activateur plasminogène du tissu amélioré selon la revendication 1.

4. Un procédé pour préparer un activateur plasminogène du tissu amélioré selon la revendication 1 qui comprend l'expression du codage ADN dudit activateur plasminogène du tissu amélioré dans un hôte approprié et la récupération de l'activateur plasminogène du tissu amélioré exprimé.

5. Un procédé selon la revendication 4 dans lequel l'hôte est des cellules CHO.

Fig. 1 (part 1)

1. 71MER 5' GATCTATGGATGCAATGAAGAGAG
GGCTCTGCTGTGTGCTGCTACTCT
GCGGAGCAGTCTTCGTTTCGCCC 3'

2. 48MER 5' AGCCAGGAAATCCATGCCCGATTC
AGAAGAGGAGCCAGGTCTTACCAA 3'

3. 55MER 5' GACTGCTCCGCAGAGTAGCAGCAC
ACAGCAGAGCCCTCTCTTCATTGC
ATCCATA 3'

4. 644MER 5' TCACTTGGTAAGACCTGGCTCCTC
TTCTGAATCGGGCATGGATTTCCT
GGCTGGGCGAAACGAA 3'

5. 67MER 5' GTGATCTGCAGAGATGAAAAAACG
CAGATGATATACCAGCAACATCAG
TCATGGCTGCGCCCTGTGC 3'

6. 61MER 5' TCAGAAGCAACCGGGTGGAATATT
GCTGGTGCAACAGTGGCAGGGCAC
AGTGCCACTCAGT 3'

7. 53MER 5' GCCTGTCAAAAGTTGCAGCGAGCC
AAGGTGTTTCAACGGGGGCACCTG
CCAGC 3'

7'.54MER 5' GCCTGTCAAAAGTTGCAGCGAGCC
AAGGTGTTTCAACGGGGGCACCTG
CCAGCA 3'

8. 51MER 5' GCCATGACTGATGTTGCTGGTATA
TCATCTGCGTTTTTTCATCTCT
GC
AGA 3'

Fig. 1 (part 2)

9. 61MER 5' TGTGCCCTGCCACTGTTGCACCAG
CAATATTCCACCCGGTTGCTTCTG
AGCACAGGGCGCA 3'

10. 70MER 5' AGCTTGCTGGCAGGTGCCCCCGTT
GAAACACCTTGGCTCGCTGCAACT
TTTGACAGGCACTGAGTGGCAC 3'

11. 68MER 5' AAGCTTTGTACTTCTCAGATTTCG
TGTGCCAGTGCCCCGAAGGATTTG
CTGGGAAGTGCTGTGAAATA 3'

11'. 67MER 5' AGCTTTGTACTTCTCAGATTTCGT
GTGCCAGTGCCCCGAAGGATTTGC
TGGGAAGTGCTGTGAAATA 3'

12. 49MER 5' GATACTCGAGCCACGTCTGAGGGA
AACAGTGACTGCTACTTTGGGAAT
G 3'

13. 59MER 5' GGTCAGCCTACCGTGGTACCCACA
GCCTCACCGAGTCGGGTGCCTCCT
GCCTCCCATGG 3'

14. 50MER 5' TTCCAGCAAATCCTTCGGGGCAC
TGGCACACGAAATCTGAGAAGTAC
AA 3'

15. 74MER 5' GGTAGGCTGACCCATTCCCAAAGT
AGCAGTCACTGTTTCCCTCAGACG
TGGCTCGAGTATCTATTTCACAGC
AC 3'

16. 51MER 5' AATTCCATGGGAGGCAGGAGGCAC
CCGACTCGGTGAGGCTGTGGGTAC
CAC 3'

Fig. 2 (part 1)

5' GATCTATGGATGCAATGAAGAGAGGGCTCT

ATACCTACGTTACTTCTCTCCCGAGA

GCTGTGTGCTGCTACTCTGCGGAGCAGTCT

CGACACGACGATGAGACGCCTCGTCAGA

TCGTTTCGCCCAGCCAGGAAATCCATGCCC

AGCAAAGCGGGTCGGTCCTTTAGGTACGGG

GATTCAGAAGAGGAGCCAGGTCTTACCAAG

CTAAGTCTTCTCCTCGGTCCAGAATGGTTC

TGATCTGCAGAGATGAAAAACGCAGATGA

ACTAGACGTCTCTACTTTTTTGCGTCTACT

TATACCAGCAACATCAGTCATGGCTGCGCC

ATATGGTCGTTGTAGTCAGTACCGACGCGG

CTGTGCTCAGAAGCAACCGGGTGGAATATT

GACACGAGTCTTCGTTGGCCCACCTTATAA

GCTGGTGCAACAGTGGCAGGGCACAGTGCC

CGACCACGTTGTCACCGTCCCGTGTCACGG

ACTCAGTGCCTGTCAAAAGTTGCAGCGAGC

TGAGTCACGGACAGTTTTCAACGTCGCTCG

CAAGGTGTTTCAACGGGGGCACCTGCCAGC

GTTCCACAAAGTTGCCCCCGTGGACGGTCG

AAGCTTTGTACTTCTCAGATTTCGTGTGCC

TTCGAAACATGAAGAGTCTAAAGCACACGG

AGTGCCCCGAAGGATTTGCTGGGAAGTGCT

TCACGGGGCTTCCTAAACGACCCTTCACGA

GTGAAATAGATACTCGAGCCACGTCTGAGG

CACTTTATCTATGAGCTCGGTGCAGACTCC

Fig. 2 (part 2)

```
G A A A C A G T G A C T G C T A C T T T G G G A A T G G G T
C T T T G T C A C T G A C G A T G A A A C C C T T A C C C A
C A G C C T A C C G T G G T A C C C A C A G C C T C A C C G
G T C G G A T G G C A C C A T G G G T G T C G G A G T G G C
A G T C G G G T G C C T C C T G C C T C C C A T G G      3'
T C A G C C C A C G G A G G A C G G A G G G T A C C T T A A 5'
```

Fig. 3-1

EP 0 386 240 B1

Fig. 3-2

BglⅡ                                                    EcoRI

Block IV

M l3mp l8 ( digestion with Bam HI/EcoRI )

T4DNA ligase

Determination and confirmation of DNA base sequence

double stranded (replicative) M13mp18 (containing Block IV)

BglⅡ                                                    EcoRI

digestion with
Xho II/EcoRI

M l3mp l9 ( digestion with BamHI/EcoRI )

T4 DNA ligase

Determination and confirmation of DNA base sequence

double stranded (replicative) M13mp 19 (containing Block IV)

BglⅡ                                                    EcoRI

digestion with
XhoII/EcoRI

Fig. 4

Fig. 5-1 (part 1)

```
5' ATGGATGCAATGAAGAGAGGGCTCTGCTGT

    GTGCTGCTACTCTGCGGAGCAGTCTTCGTT

    TCGCCCAGCCAGGAAATCCATGCCCGATTC

    AGAAGAGGAGCCAGGTCTTACCAAGTGATC

    TGCAGAGATGAAAAAACGCAGATGATATAC

    CAGCAACATCAGTCATGGCTGCGCCCTGTG

    CTCAGAAGCAACCGGGTGGAATATTGCTGG

    TGCAACAGTGGCAGGGCACAGTGCCACTCA

    GTGCCTGTCAAAAGTTGCAGCGAGCCAAGG

    TGTTTCAACGGGGGCACCTGCCAGCAAGCT

    TTGTACTTCTCAGATTTCGTGTGCCAGTGC

    CCCGAAGGATTTGCTGGAAGTGCTGTGAA

    ATAGATACTCGAGCCACGTCTGAGGGAAAC

    AGTGACTGCTACTTTGGGAATGGGTCAGCC

    TACCGTGGTACCCACAGCCTCACCGAGTCG

    GGTGCCTCCTGCCTCCCATGGAATTCCATG

    ATCCTGATAGGCAAGGTTTACACAGCACAG

    AACCCCAGTGCCCAGGCACTGGGCCTGGGC

    AAACATAATTACTGCCGGAATCCTGATGGG
```

Fig. 5-1 (part 2)

```
GATGCCAAGCCCTGGTGCCACGTGCTGAAG
AACCGCAGGCTGACGTGGGAGTACTGTGAT
GTGCCCTCCTGCTCCACCTGCGGCCTGAGA
CAGTACAGCCAGCCTCAGTTTCGCATCAAA
GGAGGGCTCTTCGCCGACATCGCCTCCCAC
CCCTGGCAGGCTGCCATCTTTGCCAAGCAC
AGGAGGTCGCCCGGAGAGCGGTTCCTGTGC
GGGGGCATACTCATCAGCTCCTGCTGGATT
CTCTCTGCCGCCCACTGCTTCCAGGAGAGG
TTTCCGCCCCACCACCTGACGGTGATCTTG
GGCAGAACATACCGGGTGGTCCCTGGCGAG
GAGGAGCAGAAATTTGAAGTCGAAAAATAC
ATTGTCCATAAGGAATTCGATGATGACACT
TACGACAATGACATTGCGCTGCTGCAGCTG
AAATCGGATTCGTCCCGCTGTGCCCAGGAG
AGCAGCGTGGTCCGCACTGTGTGCCTTCCC
CCGGCGGACCTGCAGCTGCCGGACTGGACG
GAGTGTGAGCTCTCCGGCTACGGCAAGCAT
GAGGCCTTGTCTCCTTTCTATTCGGAGCGG
CTGAAGGAGGCTCATGTCAGACTGTACCCA
TCCAGCCGCTGCACATCACAACATTTACTT
AACAGAACAGTCACCGACAACATGCTGTGT
```

Fig. 5-1 (part 3)

G C T G G A G A C A C T C G G A G C G G C G G G C C C C A G

G C A A A C T T G C A C G A C G C C T G C C A G G G C G A T

T C G G G A G G C C C C C T G G T G T G T C T G A A C G A T

G G C C G C A T G A C T T T G G T G G G C A T C A T C A G C

T G G G G C C T G G G C T G T G G A C A G A A G G A T G T C

C C G G G T G T G T A C A C C A A G G T T A C C A A C T A C

C T A G A C T G G A T T C G T G A C A A C A T G C G A C C G

T G A   3'

[wherein A is deoxyadenyl, G is deoxyguanyl,

C is deoxycytidyl and T is deoxythymidyl]

Fig. 5-2 (part 1)

5' ATGGATGCAATGAAGAGAGGGCTCTGCTGT

GTGCTGCTACTCTGCGGAGCAGTCTTCGTT

TCGCCCAGCCAGGAAATCCATGCCCGATTC

AGAAGAGGAGCCAGGTCTTACCAAGTGATC

TGCAGAGATGAAAAAACGCAGATGATATAC

CAGCAACATCAGTCATGGCTGCGCCCTGTG

CTCAGAAGCAACCGGGTGGAATATTGCTGG

TGCAACAGTGGCAGGGCACAGTGCCACTCA

GTGCCTGTCAAAAGTTGCAGCGAGCCAAGG

TGTTTCAACGGGGGCACCTGCCAGCAAGCT

TTGTACTTCTCAGATTTCGTGTGCCAGTGC

CCCGAAGGATTTGCTGGGAAGTGCTGTGAA

ATAGATACTCGAGCCACGTCTGAGGGAAAC

AGTGACTGCTACTTTGGGAATGGGTCAGCC

TACCGTGGTACCCACAGCCTCACCGAGTCG

GGTGCCTCCTGCCTCCCATGGAATTCCATG

ATCCTGATAGGCAAGGTTTACACAGCACAG

AACCCCAGTGCCCAGGCACTGGGCCTGGGC

AAACATAATTACTGCCGGAATCCTGATGGG

Fig. 5-2 (part 2)

GATGCCAAGCCCTGGTGCCACGTGCTGAAG

AACCGCAGGCTGACGTGGGAGTACTGTGAT

GTGCCCTCCTGCTCCACCTGCGGCCTGAGA

CAGTACAGCCAGCCTCAGTTTCGCATCATA

GGAGGGCTCTTCGCCGACATCGCCTCCCAC

CCCTGGCAGGCTGCCATCTTTGCCAAGCAC

AGGAGGTCGCCCGGAGAGCGGTTCCTGTGC

GGGGGCATACTCATCAGCTCCTGCTGGATT

CTCTCTGCCGCCCACTGCTTCCAGGAGAGG

TTTCCGCCCCACCACCTGACGGTGATCTTG

GGCAGAACATACCGGGTGGTCCCTGGCGAG

GAGGAGCAGAAATTTGAAGTCGAAAAATAC

ATTGTCCATAAGGAATTCGATGATGACACT

TACGACAATGACATTGCGCTGCTGCAGCTG

AAATCGGATTCGTCCCGCTGTGCCCAGGAG

AGCAGCGTGGTCCGCACTGTGTGCCTTCCC

CCGGCGGACCTGCAGCTGCCGGACTGGACG

GAGTGTGAGCTCTCCGGCTACGGCAAGCAT

GAGGCCTTGTCTCCTTTCTATTCGGAGCGG

CTGAAGGAGGCTCATGTCAGACTGTACCCA

TCCAGCCGCTGCACATCACAACATTTACTT

AACAGAACAGTCACCGACAACATGCTGTGT

Fig. 5-2 (part 3)

G C T G G A G A C A C T C G G A G C G G C G G G C C C C A G

G C A A A C T T G C A C G A C G C C T G C C A G G G C G A T

T C G G G A G G C C C C C T G G T G T G T C T G A A C G A T

G G C C G C A T G A C T T T G G T G G G C A T C A T C A G C

T G G G G C C T G G G C T G T G G A C A G A A G G A T G T C

C C G G G T G T G T A C A C C A A G G T T A C C A A C T A C

C T A G A C T G G A T T C G T G A C A A C A T G C G A C C G

T G A  3'

[wherein A is deoxyadenyl, G is deoxyguanyl,

C is deoxycytidyl and T is deoxythymidyl]

Fig. 6-1 (part 1)

H₂N — Met Asp Ala Met Lys Arg Gly Leu Cys Cys

Val Leu Leu Leu Cys Gly Ala Val Phe Val

Ser Pro Ser Gln Glu Ile His Ala Arg Phe

Arg Arg Gly Ala Arg Ser Tyr Gln Val Ile

Cys Arg Asp Glu Lys Thr Gln Met Ile Tyr

Gln Gln His Gln Ser Trp Leu Arg Pro Val

Leu Arg Ser Asn Arg Val Glu Tyr Cys Trp

Cys Asn Ser Gly Arg Ala Gln Cys His Ser

Val Pro Val Lys Ser Cys Ser Glu Pro Arg

Cys Phe Asn Gly Gly Thr Cys Gln Gln Ala

Leu Tyr Phe Ser Asp Phe Val Cys Gln Cys

Pro Glu Gly Phe Ala Gly Lys Cys Cys Glu

Ile Asp Thr Arg Ala Thr Ser Glu Gly Asn

Ser Asp Cys Tyr Phe Gly Asn Gly Ser Ala

Tyr Arg Gly Thr His Ser Leu Thr Glu Ser

Gly Ala Ser Cys Leu Pro Trp Asn Ser Met

Ile Leu Ile Gly Lys Val Tyr Thr Ala Gln

Asn Pro Ser Ala Gln Ala Leu Gly Leu Gly

Lys His Asn Tyr Cys Arg Asn Pro Asp Gly

Fig. 6-1 (part 2 )

Asp Ala Lys Pro Trp Cys His Val Leu Lys

Asn Arg Arg Leu Thr Trp Glu Tyr Cys Asp

Val Pro Ser Cys Ser Thr Cys Gly Leu Arg

Gln Tyr Ser Gln Pro Gln Phe Arg Ile Lys

Gly Gly Leu Phe Ala Asp Ile Ala Ser His

Pro Trp Gln Ala Ala Ile Phe Ala Lys His

Arg Arg Ser Pro Gly Glu Arg Phe Leu Cys

Gly Gly Ile Leu Ile Ser Ser Cys Trp Ile

Leu Ser Ala Ala His Cys Phe Gln Glu Arg

Phe Pro Pro His His Leu Thr Val Ile Leu

Gly Arg Thr Tyr Arg Val Val Pro Gly Glu

Glu Glu Gln Lys Phe Glu Val Glu Lys Tyr

Ile Val His Lys Glu Phe Asp Asp Asp Thr

Tyr Asp Asn Asp Ile Ala Leu Leu Gln Leu

Lys Ser Asp Ser Ser Arg Cys Ala Gln Glu

Ser Ser Val Val Arg Thr Val Cys Leu Pro

Pro Ala Asp Leu Gln Leu Pro Asp Trp Thr

Glu Cys Glu Leu Ser Gly Tyr Gly Lys His

Glu Ala Leu Ser Pro Phe Tyr Ser Glu Arg

Leu Lys Glu Ala His Val Arg Leu Tyr Pro

Ser Ser Arg Cys Thr Ser Gln His Leu Leu

Asn Arg Thr Val Thr Asp Asn Met Leu Cys

Fig. 6-1 (part 3)

Ala Gly Asp Thr Arg Ser Gly Gly Pro Gln

Ala Asn Leu His Asp Ala Cys Gln Gly Asp

Ser Gly Gly Pro Leu Val Cys Leu Asn Asp

Gly Arg Met Thr Leu Val Gly Ile Ile Ser

Trp Gly Leu Gly Cys Gly Gln Lys Asp Val

Pro Gly Val Tyr Thr Lys Val Thr Asn Tyr

Leu Asp Trp Ile Arg Asp Asn Met Arg Pro -COOH

[wherein $H_2N$ is amino terminal and

-COOH is carboxy terminal]

Fig. 6-2 (part 1)

```
H₂M — Met Asp Ala Met Lys Arg Gly Leu Cys Cys
      Val Leu Leu Leu Cys Gly Ala Val Phe Val
      Ser Pro Ser Gln Glu Ile His Ala Arg Phe
      Arg Arg Gly Ala Arg Ser Tyr Gln Val Ile
      Cys Arg Asp Glu Lys Thr Gln Met Ile Tyr
      Gln Gln His Gln Ser Trp Leu Arg Pro Val
      Leu Arg Ser Asn Arg Val Glu Tyr Cys Trp
      Cys Asn Ser Gly Arg Ala Gln Cys His Ser
      Val Pro Val Lys Ser Cys Ser Glu Pro Arg
      Cys Phe Asn Gly Gly Thr Cys Gln Gln Ala
      Leu Tyr Phe Ser Asp Phe Val Cys Gln Cys
      Pro Gln Gly Phe Ala Gly Lys Cys Cys Glu
      Ile Asp Thr Arg Ala Thr Ser Glu Gly Asn
      Ser Asp Cys Tyr Phe Gly Asn Gly Ser Ala
      Tyr Arg Gly Thr His Ser Leu Thr Glu Ser
      Gly Ala Ser Cys Leu Pro Trp Asn Ser Met
      Ile Leu Ile Gly Lys Val Tyr Thr Ala Gln
      Asn Pro Ser Ala Gln Ala Leu Gly Leu Gly
      Lys His Asn Tyr Cys Arg Asn Pro Asp Gly
```

Fig. 6-2 (part 2)

Asp Ala Lys Pro Trp Cys His Val Leu Lys

Asn Arg Arg Leu Thr Trp Glu Tyr Cys Asp

Val Pro Ser Cys Ser Thr Cys Gly Leu Arg

Gln Tyr Ser Gln Pro Gln Phe Arg Ile Ile

Gly Gly Leu Phe Ala Asp Ile Ala Ser His

Pro Trp Gln Ala Ala Ile Phe Ala Lys His

Arg Arg Ser Pro Gly Glu Arg Phe Leu Cys

Gly Gly Ile Leu Ile Ser Ser Cys Trp Ile

Leu Ser Ala Ala His Cys Phe Gln Glu Arg

Phe Pro Pro His His Leu Thr Val Ile Leu

Gly Arg Thr Tyr Arg Val Val Pro Gly Glu

Glu Glu Gln Lys Phe Glu Val Glu Lys Tyr

Ile Val His Lys Glu Phe Asp Asp Asp Thr

Tyr Asp Asn Asp Ile Ala Leu Leu Gln Leu

Lys Ser Asp Ser Ser Arg Cys Ala Gln Glu

Ser Ser Val Val Arg Thr Val Cys Leu Pro

Pro Ala Asp Leu Gln Leu Pro Asp Trp Thr

Glu Cys Glu Leu Ser Gly Tyr Gly Lys His

Glu Ala Leu Ser Pro Phe Tyr Ser Glu Arg

Leu Lys Glu Ala His Val Arg Leu Tyr Pro

Ser Ser Arg Cys Thr Ser Gln His Leu Leu

Asn Arg Thr Val Thr Asp Asn Met Leu Cys

43

Fig. 6-2 (part 3)

Ala Gly Asp Thr Arg Ser Gly Gly Pro Gln

Ala Asn Leu His Asp Ala Cys Gln Gly Asp

Ser Gly Gly Pro Leu Val Cys Leu Asn Asp

Gly Arg Met Thr Leu Val Gly Ile Ile Ser

Trp Gly Leu Gly Cys Gly Gln Lys Asp Val

Pro Gly Val Tyr Thr Lys Val Thr Asn Tyr

Leu Asp Trp Ile Arg Asp Asn Met Arg Pro -COOH

[wherein H$_2$N-  is amino terminal and

-COOH is carboxy terminal ]

Fig. 7

Fig. 8

Fig. 9

Fig. 10

● Natural t-PA

○ t-PA [Ⅵ]

EP 0 386 240 B1

Fig. 11

Fig. 12

Fig. 13

Activation of improved t-PA with denatured protein

Y-axis: Amidolytic Activity ($\Delta$OD405)

X-axis: Improved t-PA[VI] activator (µg protein/ml)

BrCN-treated fibrinogen

NaOH-treated albumin

HCl-treated IgG

Intact IgG
Intact albumin

EP 0 386 240 B1

Fig. 14

Degradation of denatured protein with improved t-PA

|   |   |
|---|---|
| 1 ～ 4 : | prior to reacting with improved t-PA |
| 5 ～ 8 : | after reacting with improved t-PA |
| 1 , 5 : | intact albumin |
| 2 , 6 : | alkali-treated albumin |
| 3 , 7 : | intact IgG |
| 4 , 8 : | alkali-treated IgG |

|   |   |
|---|---|
| BSA: | calf serum albumin |
| IgG − H: | IgG H-chain |
| IgG − L: | IgG L-chain |
| — : | decomposition product |